# EUROPEAN PATENT SPECIFICATION

(11) **EP 2 501 285 B1**
(45) Date of publication and mention of the grant of the patent: **10.01.2018**
(21) Application number: 10782787.5
(22) Date of filing: 19.11.2010
(51) Int. Cl.: A61B 5/145, H01B 7/08

(54) **MULTI-CONDUCTOR LEAD CONFIGURATIONS USEFUL WITH MEDICAL DEVICE SYSTEMS**
MULTILEITER-ELEKTRODENKONFIGURATIONEN FÜR MEDIZINISCHE GERÄTESYSTEME
CONFIGURATIONS DE FILS MULTICONDUCTEURS UTILES AVEC DES SYSTÈMES DE DISPOSITIFS MÉDICAUX

(30) Priority: 20.11.2009 US 263068 P; 18.11.2010 US 949038
(43) Date of publication of application: 26.09.2012
(73) Proprietor: Medtronic MiniMed, Inc., Northridge, CA 91329-1219 (US)
(72) Inventor: SHAH, Rajiv, Rancho Palos Verdes, California 90275 (US); GOTTLIEB, Rebecca K., Culver City, California 90232 (US); WOLFE, Katherine T., Dunwoody, Georgia 30338 (US)
(74) Representative: Ruschke, Hans Edvard
(86) International application number: PCT/US2010/057474
(87) International publication number: WO 2011/063259

(56) References cited:
- EP-A1- 0 089 541
- EP-A1- 0 092 797
- WO-A1-2005/048834
- WO-A1-2009/023017
- DE-A1- 3 300 246
- DE-U1- 8 322 828
- US-A- 2 155 060
- US-A- 5 552 565
- US-A- 5 928 228
- US-A1- 2004 236 201
- US-B1- 6 295 476
- HETKE J F ET AL: "Silicon ribbon cables for chronically implantable microelectrode arrays", IEEE TRANSACTIONS ON BIOMEDICAL ENGINEERING, IEEE SERVICE CENTER, PISCATAWAY, NJ, USA, vol. 41, no. 4, 1 April 1994 (1994-04-01), pages 314-321, XP002238344, ISSN: 0018-9294, DOI: DOI:10.1109/10.284959

## Description

### Cross Reference To Related Applications

This application claims priority from U.S. Provisional Application Serial No. 61/263,068 filed November 20, 2009, and U.S. Patent Application Serial No. 12/949,038 filed November 18, 2010, the contents of which are incorporated herein by reference. This application is related to U.S. Patent Application Serial No. 11/492,273, U.S. Patent Application Serial No. 11/633,254, U.S. Patent Application Serial No 12/184,046, U.S. Patent Application No. 12/345,354, and U.S. Patent Application No. 12/572,087, the contents of each of which are herein incorporated by reference.

### Background of the Invention

### 1. Field of the Invention.

This invention relates to medical devices such as glucose sensors used in the management of diabetes and electronic leads used with such devices.

### 2. Description of Related Art.

A wide variety of medical devices are known in the art, for example analyte sensors. Common analyte sensors include devices that use biological elements to convert a chemical analyte in a matrix into a detectable signal. There are many types of biosensors used to detect a wide variety of analytes. Perhaps the most studied type of biosensor is the amperometric glucose sensor, an apparatus commonly used to monitor glucose levels in individuals with diabetes.

A typical glucose sensor works according to the following chemical reactions:

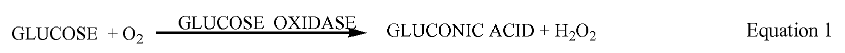

H₂O₂ O₂+2H⁺ + 2e⁻ Equation 2

The glucose oxidase in such sensors is used to catalyze the reaction between glucose and oxygen to yield gluconic acid and hydrogen peroxide as shown in equation 1. The H₂O₂ reacts electrochemically as shown in equation 2, and the current is measured by a potentiostat. These reactions, which occur in a variety of oxidoreductases known in the art, are used in a number of sensor designs.

As medical device technology matures and new applications for this technology are developed, there is a need for elements that facilitate the use of devices such as analyte sensors in the wide variety of situations in which the use of such devices is desirable. US-2004/236201-A1 shows an implantable glucose sensor with a coiled ribbon cable. WO-2005/048834-A1 shows an implantable glucose sensor array.

### Summary of the Invention

The invention is defined by the claims. Embodiments of the invention disclosed herein include medical device systems such as amperometric glucose sensor systems used in the management of diabetes and optimized elements for use with such device systems. Typical embodiments of the invention include a medical device that is operatively coupled to a multi-conductor electrical lead having a coiled configuration as disclosed herein. The compact architecture of the multi-conductor lead designs disclosed herein allows various elements in medical device systems to be electrically connected together in a space saving configuration, one that optimizes the use of such systems in a variety of contexts including situations where a patient is ambulatory and outside of a clinical environment, as well as conventional hospital environments.

Illustrative embodiments of the invention include medical device systems comprising a coiled conductor design where multiple conductive elements such as wires disposed within a ribbon cable are wrapped around a central core element in an arrangement that minimizes the space required for electrical leads used to operatively connect one element in the system to another. Illustrative medical device systems that use such multiple-conductor electrical leads include a variety of analyte sensor apparatuses, for example one comprising a sensor having a base layer; a conductive layer disposed on the base layer and comprising a reference electrode, a working electrode and a counter electrode; an analyte sensing layer (e.g. one comprising glucose oxidase) disposed on the conductive layer; an analyte modulating layer disposed on the analyte sensing layer; and a cover layer having an aperture disposed on the analyte sensor apparatus. Typically, such analyte sensors are operatively coupled to a sensor input capable of receiving a signal from the sensor that is based on a sensed analyte; and a processor coupled to the sensor input, wherein the processor is capable of characterizing one or more signals received from the sensor. In some embodiments of the invention, a medical device system that uses a multiple-conductor electrical lead as disclosed herein includes electronic components designed to transmit and/or receive and/or display signal data (e.g. monitors and the like) and/or devices that can use data obtained from such sensor systems to modulate a patient's physiology (e.g. medication infusion pumps).

One embodiment of the invention comprises a multiple-conductor electrical lead having a central core element; and one or more electrical conduits (e.g. in the form of a ribbon cable) coiled around the central core along a length of the central core. Typically, the electrical conduit is disposed in a ribbon cable having one or more (typically a plurality) separate electrical conduits coupled together along their lengths in series and electrically insulated from one another with an insulating material. In a specific illustrative embodiment, the multiple-conductor electrical lead comprises 1, 2, 3, 4, 5, 6 or more electrical conduits coiled around a core central strand or fibril, one that is typically made from a material selected to provide flexibility and/or tensile strength to the lead (e.g. a polyester strand). This central strand or core element can comprise a single fiber or filament, or alternatively a plurality of fibers or filaments that are intertwined, so that this core element forms a flexible central strand around which conductive elements (e.g. ribbon cables) are wrapped in a space saving configuration.

The central core element of the multi-conductor leads disclosed herein can be made from one or more compositions in order to control the material properties of the multiple-conductor electrical lead. For example, in some embodiments of the invention, the core can comprise an elastic fiber such as a fiber made from a polymeric composition (e.g. a polyester compound, a thermoplastic polyamide compound or the like). In some embodiments, the core comprises a metallic composition, for example a stainless steel composition or a nickel titanium composition having elastic properties. While many conductive metals can be used as electrical conduits in embodiments of the invention, the flexible stability of the multiple-conductor electrical leads disclosed herein allows for the greater use of noble metal conductors (e.g. platinum, gold, silver, iridium and copper). Consequently, in certain embodiments of the invention, the multi-conductor lead comprises a noble metal conductor and is designed so that the conductor is coupled to and supported by the flexible central core in a manner that decreases problems associated with the inflexibility and/or fragility of certain noble metal conductor compositions.

One key feature of the multi-conductor electrical lead design disclosed herein is that it is scalable, and is not, for example, limited to a specific number of electrical conduits and/or ribbon cables. For example, embodiments of the invention can comprise one or more ribbon cables wrapped around a primary central core element, with this primary coiled structure then functioning as a central core element around which further ribbon cables are wrapped to form a secondary coiled structure. In an illustration of this, embodiments of the invention can comprise 1, 2, 3, 4 or more ribbon cables coiled around a flexible central strand, with this structure then functioning as a core element that itself then has 1, 2, 3, 4 or more further ribbon cables wrapped around it. One such embodiment comprises a 4 conductor ribbon cable wrapped around a flexible polyester cord to form a primary coiled structure, with two 6 conductor ribbon cables then being wrapped around this primary coiled structure (which then functions as a core element) to form a secondary coiled structure. In addition, in certain embodiments of the invention, a secondary coiled structure can itself function as a core element around which further ribbon cables are wrapped to form a tertiary coiled structure. In this manner, the various configurations of the leads disclosed herein optimize the use of space.

In the coiled multi-conductor electrical lead design disclosed herein, factors such as cable wrapping pitch and cable wrapping tension can be controlled in a manner that stabilizes the lead components and/or optimizes the space saving architecture of the multi-conductor lead. For example, in certain embodiments of the invention, the wrapping pitch of a cable around a central core is set so a single ribbon cable is coiled around the central core such that a substantially uniform gap is formed between coils of the single ribbon cable. In other embodiments of the invention, the wrapping pitch of a cable around a central core is set so as to abut an already coiled ribbon cable while avoiding an overlap with this coiled ribbon cable. Alternatively, the wrapping pitch of a cable around a central core is set so that with each consecutive wrapping, the cable is wrapped around the core so as to overlap with an existing cable wrapping by a ¼, ½ or ¾ width. In addition, it is possible to optimize the space saving architecture of adjacent cables throughout the entire lead structure by adjusting factors including ribbon cable width, the amount of ribbon cable overlap (if any) and the ribbon cable wrapping tension. The choice and range of these parameters can be used to properly balance lead conductivity, flexibility and stability, for example by adjusting the diameter of the core elements, the compositions of these elements, the number of electrical conduits and/or electrical cables, ribbon cable spacing and ribbon cable tension forces. In addition, certain embodiments of the invention can include one or more materials incorporated into and/or disposed around the multi-conductor electrical leads, in order to, for example, adhere, support and/or electrically shield one or more elements of the multi-conductor electrical lead (e.g. an adhesive material or the like, an electrically non-conducting shielding material or the like etc.).

While amperometric glucose sensors are discussed as a typical device used with the multi-conductor electrical leads disclosed herein, these leads can be used with a variety of different medical devices in a wide variety of contexts/applications. However, embodiments of the multi-conductor lead design are particularly useful in device applications that involve DC potential, for example DC biased sensing applications. In some applications, the medical device used with this lead (e.g. an amperometric glucose sensor) is operatively coupled to one or more elements designed for use in ambulatory contexts such as a flex-circuit. One such illustrative sensor flex-circuit embodiment has two columns of contact pads on the left with the electrodes on the right, wherein the close proximity of the pads in this design allows for ease of connection to a multi-conductor lead as well as a compact design.

As noted above, the multi-conductor lead configurations disclosed herein are compact and very space efficient. The space saving configuration provides a number of desirable properties and, for example can reduce the amount of trauma that occurs at an *in vivo* implantation site (e.g. the implantation site of an electrochemical glucose sensor of the type used in the management of diabetes). Consequently, embodiments of the invention include methods for decreasing the degree of tissue trauma at the site where a medical device is implanted *in vivo*, the method comprising supplying an electrical signal to the implanted device using an embodiment of the multi-conductor lead design configurations disclosed herein. In illustrative embodiments of this method, the implanted device is an amperometric glucose sensor. Similarly, in certain embodiments, multi-conductor lead embodiments of the invention allow an implanted device (e.g. a glucose sensor) to operate at a location that is distal (farther away) from a site of implantation than is typically possible using conventional electronic leads. Consequently, embodiments of the invention include methods for selecting a location where a medical device is implanted *in vivo*, the method comprising selecting a distal site as one both compatible with a multi-conductor lead design configuration as well as being optimized for user comfort and then supplying power to the implanted to device using an embodiment of the multi-conductor lead design configurations disclosed herein. Certain embodiments of the invention include methods of using a specific multi-conductor lead and device (e.g. sensor) element and/or a specific constellation of sensor elements to produce and/or facilitate one or more properties of the device (e.g. to enhance its biocompatibility profile). Typical embodiments of the invention are comprised of biocompatible materials and/or have structural elements and organizations of elements designed for implantation *in vivo*. As discussed below, other methodological embodiments of the invention include methods for making and using the multi-conductor lead embodiments disclosed herein.

Other objects, features and advantages of the present invention will become apparent to those skilled in the art from the following detailed description. It is to be understood, however, that the detailed description and specific examples, while indicating some embodiments of the present invention are given by way of illustration and not limitation. Many changes and modifications within the scope of the present invention may be made without departing from the scope of the claims, and the invention includes all such modifications.

### Brief Description of the Figures

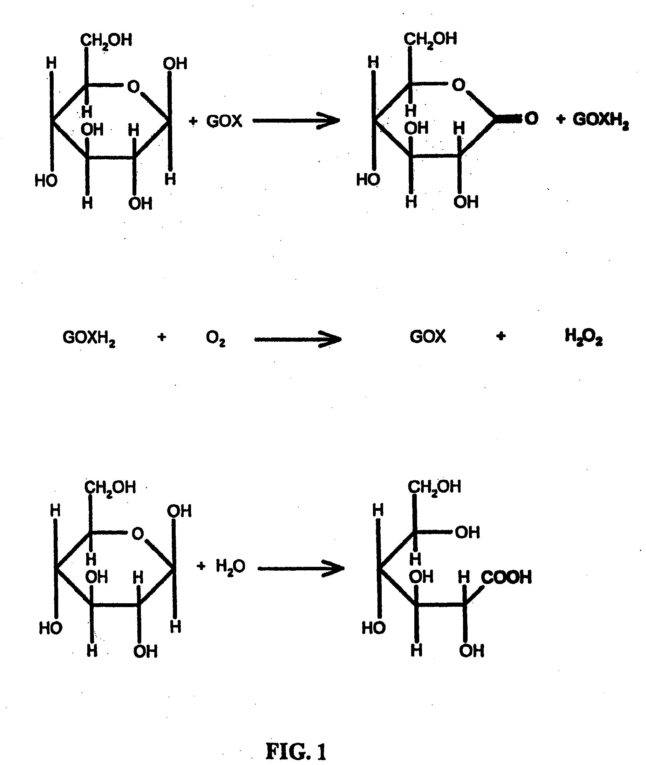

FIG. 1 provides a schematic of the well known reaction between glucose and glucose oxidase. As shown in a stepwise manner, this reaction involves glucose oxidase (GOx), glucose and oxygen in water. In the reductive half of the reaction, two protons and electrons are transferred from β-D-glucose to the enzyme yielding d-gluconolactone. In the oxidative half of the reaction, the enzyme is oxidized by molecular oxygen yielding hydrogen peroxide. The d-gluconolactone then reacts with water to hydrolyze the lactone ring and produce gluconic acid. In certain electrochemical sensors of the invention, the hydrogen peroxide produced by this reaction is oxidized at the working electrode (H₂O₂ → 2H+ + O₂ + 2e⁻).

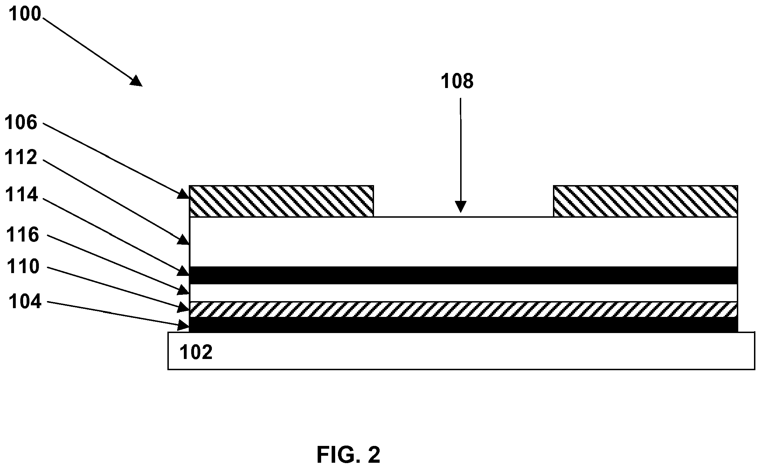

FIG. 2 provides a diagrammatic view of a typical layered analyte sensor configuration for use with embodiments of the current invention.

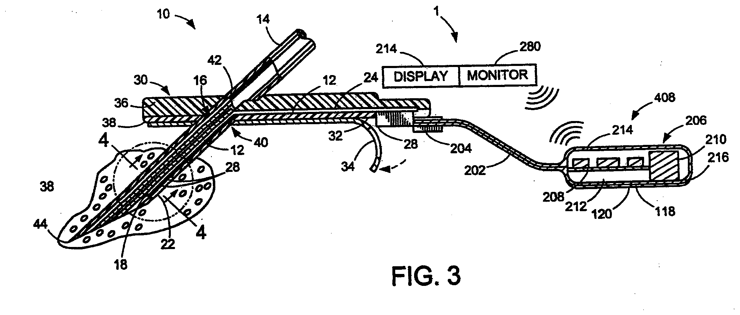

FIG. 3 provides a perspective view illustrating a subcutaneous sensor insertion set, a telemetered characteristic monitor transmitter device, and a data receiving device embodying features of the invention.

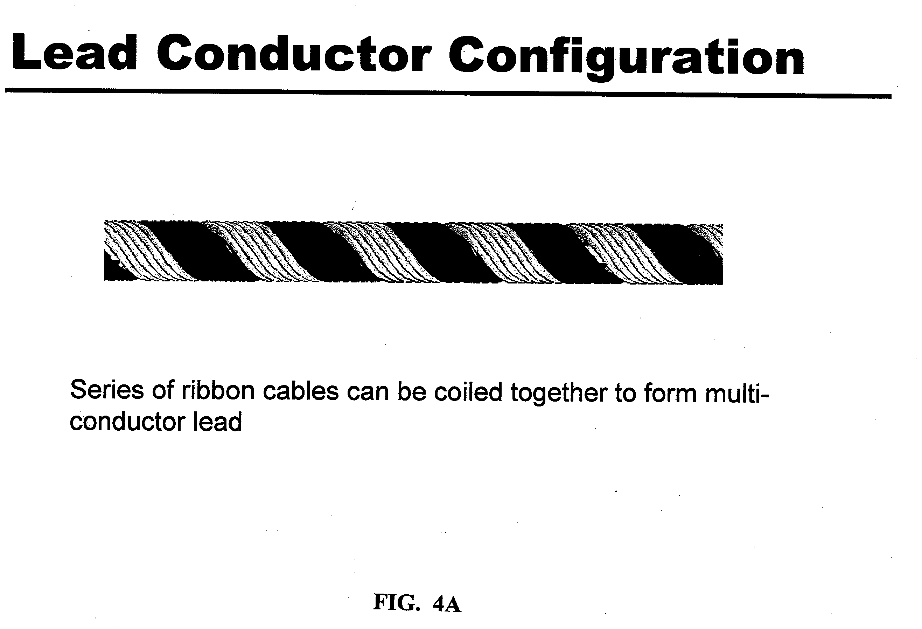

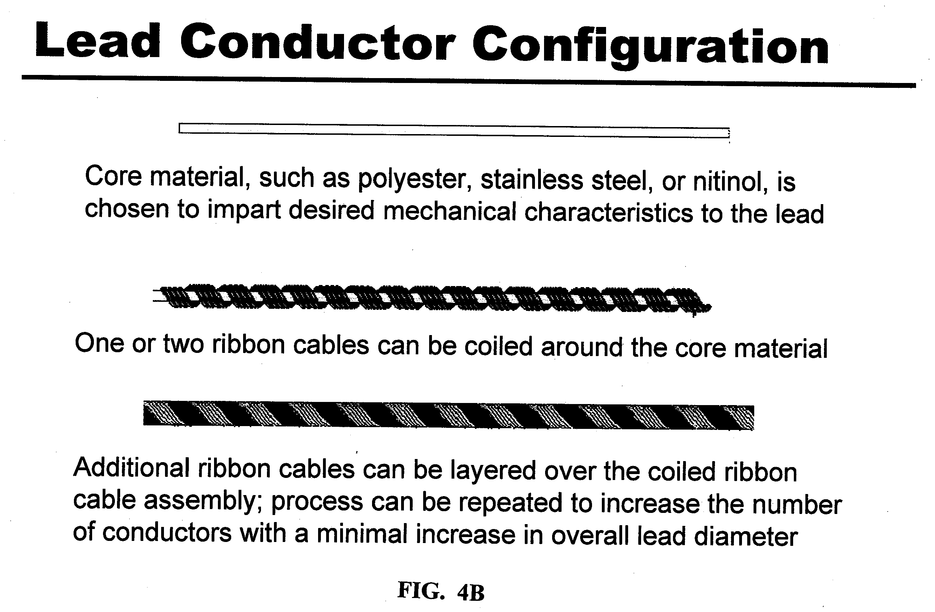

FIGS. 4A and 4B provide representations of illustrative embodiments of multi-conductor lead configurations of the invention. FIG 4A shows an embodiment of a lead conductor configuration where a series of ribbon cables is coiled together to form a multi-conductor lead. FIG. 4B shows an embodiment of a lead conductor configuration where a core material such as polyester, stainless steel of a shape memory alloy such as Nitinol® can be selected depending upon the mechanical characteristics desired for the lead. One or two (or more) ribbon materials can then be coiled around this core material. Additional ribbon cables can then be layered over this coiled ribbon cable assembly; and this process can then be repeated to, for example, increase the number of conductors within the lead with only a minimal increase in overall lead diameter.

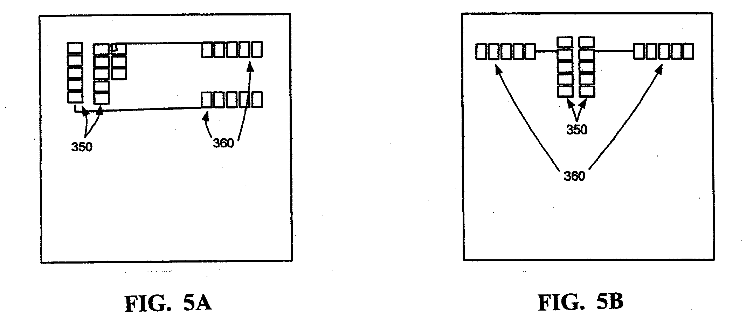

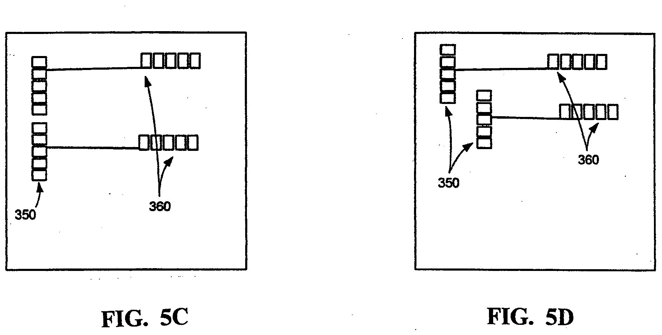

FIGS. 5A-5D provide schematics of sensor flex layouts. The embodiment shown in FIG. 5A has two columns of contact pads (350) on the left with the electrodes (360) on the right. The embodiment shown in FIG. 5B has the two columns of contact pads (350) at the center in between both sensor electrodes (360). The embodiment shown in FIG. 5C has a single column of contact pads (350) allowing for a different connection scheme with more width space than the design shown in FIG 5A. The embodiment shown in FIG. 5D shows a staggered element layout. In embodiments of the invention that comprise multiple sensors, multiple groups of one or more of these layouts can be disposed together (e.g. in a repetitive pattern).

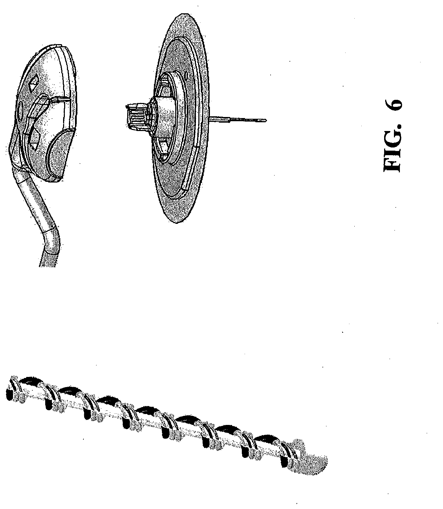

FIG. 6 discloses one of the many illustrative medical devices (a glucose sensor) and an embodiment of a multi-conductor lead useful with such devices.

### Detailed Description of the Embodiments

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted. A number of terms are defined below.

All publications mentioned herein are incorporated herein by reference to disclose and describe the methods and/or materials in connection with which the publications are cited. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

Before the present device systems and methods etc. are described, it is to be understood that this invention is not limited to the particular structure, methodology, protocol, composition etc., described as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Thus, for example, reference to "a ribbon cable" includes a plurality of such ribbon cables and equivalents thereof known to those skilled in the art, and so forth. All numbers recited in the specification and associated claims that refer to values that can be numerically characterized with a value other than a whole number are understood to be modified by the term "about".

The term "analyte" as used herein is a broad term and is used in its ordinary sense, including, without limitation, to refer to a substance or chemical constituent in a fluid such as a biological fluid (for example, blood, interstitial fluid, cerebral spinal fluid, lymph fluid or urine) that can be analyzed. Analytes can include naturally occurring substances, artificial substances, metabolites, and/or reaction products. In some embodiments, the analyte for measurement by the sensing regions, devices, and methods is glucose. However, other analytes are contemplated as well, including but not limited to, lactate. Salts, sugars, proteins fats, vitamins and hormones naturally occurring in blood or interstitial fluids can constitute analytes in certain embodiments. The analyte can be naturally present in the biological fluid or endogenous; for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin. The metabolic products of drugs and pharmaceutical compositions are also contemplated analytes.

The term "oxidoreductase" is used according to its art accepted meaning, i.e. an enzyme that catalyzes the transfer of electrons from one molecule (the reductant, also called the hydrogen or electron donor) to another (the oxidant, also called the hydrogen or electron acceptor). Typical oxidoreductases include glucose oxidase and lactate oxidase. The term "carrier polypeptide" or "carrier protein" is used according to its art accepted meaning of an additive included to maintain the stability of a polypeptide, for example the ability of an oxidoreductase polypeptide to maintain certain qualitative features such as physical and chemical properties (e.g. an ability to oxidize glucose) of a composition comprising a polypeptide for a period of time. A typical carrier protein commonly used in the art is albumin.

The term "sensor," as used herein, is a broad term and is used in its ordinary sense, including, without limitation, the portion or portions of an analyte-monitoring device that detects an analyte. In one embodiment, the sensor includes an electrochemical cell that has a working electrode, a reference electrode, and optionally a counter electrode passing through and secured within the sensor body forming an electrochemically reactive surface at one location on the body, an electronic connection at another location on the body, and a membrane system affixed to the body and covering the electrochemically reactive surface. During general operation of the sensor, a biological sample (for example, blood or interstitial fluid), or a portion thereof, contacts (directly or after passage through one or more membranes or domains) an enzyme (for example, glucose oxidase); the reaction of the biological sample (or portion thereof) results in the formation of reaction products that allow a determination of the analyte level in the biological sample.

The terms "electrical potential" and "potential" as used herein, are broad terms and are used in their ordinary sense, including, without limitation, the electrical potential difference between two points in a circuit which is the cause of the flow of a current. The term "system noise," as used herein, is a broad term and is used in its ordinary sense, including, without limitation, unwanted electronic or diffusion-related noise which can include Gaussian, motion-related, flicker, kinetic, or other white noise, for example.

As discussed in detail below, embodiments of the invention can comprise electrochemical sensors that measure a concentration of an analyte of interest or a substance indicative of the concentration or presence of the analyte in fluid. In some embodiments, the sensor is a continuous device, for example a subcutaneous, transdermal, or intravascular device. In some embodiments, the device can analyze a plurality of intermittent blood samples. The sensor embodiments disclosed herein can use any known method, including invasive, minimally invasive, and non-invasive sensing techniques, to provide an output signal indicative of the concentration of the analyte of interest. Typically, the sensor is of the type that senses a product or reactant of an enzymatic reaction between an analyte and an enzyme in the presence of oxygen as a measure of the analyte *in vivo* or in vitro. Such sensors typically comprise a membrane layer surrounding the enzyme through which an analyte migrates. The product is then measured using electrochemical methods and thus the output of an electrode system functions as a measure of the analyte. In some embodiments, the sensor can use an amperometric, coulometric, conductimetric, and/or potentiometric technique for measuring the analyte.

Typical embodiments of the invention disclosed herein comprise sensors of the type used, for example, in subcutaneous or transcutaneous monitoring of blood glucose levels in a diabetic patient. A variety of implantable, electrochemical biosensors have been developed for the treatment of diabetes and other life-threatening diseases. Many existing sensor designs use some form of immobilized enzyme to achieve their bio-specificity. Embodiments of the invention described herein can be adapted and implemented with a wide variety of known electrochemical sensors, including for example, U.S. Patent Application No. 20050115832, U.S. Pat. Nos. 6,001,067, 6,702,857, 6,212,416, 6,119,028, 6,400,974, 6,595,919, 6,141,573, 6,122,536, 6,512,939 5,605,152, 4,431,004, 4,703,756, 6,514,718, 5,985,129, 5,390,691, 5,391, 250, 5,482,473, 5,299,571, 5,568,806, 5,494,562, 6,120,676, 6,542,765 as well as PCT International Publication Numbers WO 01/58348, WO 04/021877, WO 03/034902, WO 03/035117, WO 03/035891, WO 03/023388, WO 03/022128, WO 03/022352, WO 03/023708, WO 03/036255, WO03/036310 WO 08/042625, and WO 03/074107, and European Patent Application EP 1153571, the contents of each of which are incorporated herein by reference.

Embodiments of the invention disclosed herein provide medical device system elements having enhanced material properties and/or architectural configurations as well as analyte sensor systems (e.g. those comprising a sensor and associated electronic components such as a lead, a monitor, a processor and the like) constructed to include such elements. The disclosure further provides methods for making and using such elements and/or architectural configurations. While some embodiments of the invention pertain to glucose and/or lactate sensors, a variety of the elements disclosed herein (e.g. multi-conductor lead designs) can be adapted for use with any one of the wide variety of medical devices known in the art. The medical device elements, architectures and methods for making and using these elements that are disclosed herein exhibit a surprising degree of flexibility and versatility, characteristics which allow their use with a wide variety of medical device systems.

Specific aspects of embodiments of the invention are discussed in detail in the following sections.

### ILLUSTRATIVE EMBODIMENTS OF THE INVENTION

The invention disclosed herein has a number of embodiments. Illustrative embodiments of the invention disclosed herein include medical device systems such as implantable amperometric glucose sensor systems used in the management of diabetes and optimized elements for use with such device systems. Typical embodiments of the invention include a medical device system that is operatively coupled to a multi-conductor electrical lead having a coiled configuration as disclosed herein (see, e.g. FIGS. 4A and 4B). The compact architecture of the multi-conductor lead designs disclosed herein allows various elements in medical device systems to be electrically connected together in a space saving configuration, one that optimizes the use of such systems in a variety of contexts including situations where a patient is ambulatory and outside of a clinical environment as well as conventional hospital environments. While sensor systems and amperometric glucose sensor systems in particular are discussed in detail in the following description, those of skill in the art will understand that the multi-conductor electrical lead disclosed herein can be used with a wide variety of medical devices known in the art.

Illustrative embodiments of the invention include an analyte sensor system comprising a coiled conductor design where one or more conductive elements such as wires are wrapped around a central core element in an arrangement that minimizes the space required for the electrical leads that used to operatively connect one element in the system to another. Typically, the conductive elements are disposed within a ribbon cable. A ribbon cable (also known as multi-wire planar cable) is a cable with many conducting wires running parallel to each other on the same flat plane. As a result the cable is wide and flat. Its name comes from the resemblance of the cable to a piece of ribbon (which is likewise wide and flat). Ribbon cables are usually specified by two numbers: the spacing or pitch of the conductors, and the number of conductors or ways. A spacing of 0.05 inch (1.27 mm) is the most usual, allowing for a two-row connector with a pin spacing of 0.1 inch (2.54 mm). These types are used for many types of equipment, in particular for interconnections within an enclosure. Finer pitches, for example 0.3 mm, are used in portable electronic equipment. Based on the type of standard connectors, the number of conductors typically encompasses a few values, including 4, 6, 8, 9, 10, 14, 15, 16, 18, 20, 24, 25, 26, 34, 37, 40, 50, 60, 64 and 80. A typical conductor comprises a stranded copper wire. However, other conductive compositions such as other noble metals (e.g. platinum, gold, silver and iridium) can be used. Ribbon cables allow mass termination to connectors, for example those in which the ribbon cable conduits (e.g. a ribbon cable having pins or the like attached to its conduit tips) connect with forked contacts. Illustrative types of connectors for ribbon cables include BT224 connectors, D-subminiature connectors, PCB transition headers and DIL headers. Ribbon cables may have one or more conductive layers with embedded conductors for electrical noise shielding.

Illustrative medical device systems that use the multiple-conductor electrical lead embodiments disclosed herein include a variety of analyte sensor apparatuses, for example one comprising a sensor having a base layer; a conductive layer disposed on the base layer and comprising a reference electrode, a working electrode and a counter electrode; an analyte sensing layer (e.g. one comprising glucose oxidase) disposed on the conductive layer; an analyte modulating layer disposed on the analyte sensing layer; and a cover layer having an aperture disposed on the analyte sensor apparatus. Typically, such analyte sensors are also operatively coupled to a sensor input capable of receiving a signal from the sensor that is based on a sensed analyte; and a processor coupled to the sensor input, wherein the processor is capable of characterizing one or more signals received from the sensor. In some embodiments of the invention, the system further includes additional elements (e.g. electronic components) such as those designed to transmit and/or receive and/or display signal data (e.g. monitors and the like) as well as devices that can use data obtained from such sensor systems to modulate a patient's physiology.

Illustrative analyte sensor systems that use such multiple-conductor electrical leads include a variety of glucose sensor apparatuses having a variety of architectural configurations. In some embodiments of the invention, an element of the sensor apparatus such as an electrode or an aperture is designed to have a specific configuration and/or is made from a specific material and/or is positioned relative to the other elements so as to facilitate a function of the sensor. In one such embodiment of the invention, a working electrode, a counter electrode and a reference electrode are positionally distributed on the base and/or the conductive layer in a configuration that facilitates sensor start up and/or maintains the hydration of the working electrode, the counter electrode and/or the reference electrode when the sensor apparatus is placed in contact with a fluid comprising the analyte (e.g. by inhibiting shadowing of an electrode, a phenomena which can inhibit hydration and capacitive start-up of a sensor circuit). Typically such embodiments of the invention facilitate glucose sensor start-up and/or initialization.

Illustrative embodiments of glucose sensor systems that can use the multiple-conductor electrical leads disclosed herein can comprise a plurality of working electrodes and/or counter electrodes and/or reference electrodes (e.g. 3 working electrodes, a reference electrode and a counter electrode), in order to, for example, provide redundant sensing capabilities. According to the invention, the plurality of working, counter and reference electrodes are configured together as a unit and positionally distributed on a conductive layer in a repeating pattern of units. According to the invention, a sensor comprises an elongated base layer is made from a flexible material that allows the sensor to twist and bend when implanted *in vivo*. In some embodiments, the electrodes are grouped in a configuration that allows the sensor to continue to function if a portion of the sensor having one or more electrodes is dislodged from *an in vivo* environment and exposed to an ex vivo environment. Certain embodiments of the invention comprising a single sensor. Other embodiments of the invention comprise multiple sensors. In some embodiments of the invention, a pulsed voltage is used to obtain a signal from one or more electrodes of a sensor.

In a sensor system embodiment of the invention that can use the multiple-conductor electrical leads and is designed to optimize electrode properties such as hydration, the working electrode, the counter electrode and the reference electrode of an amperometric sensor are positionally distributed on conductive layer in a parallel configuration arranged so that a first electrode is disposed in a region on a first edge of the elongated base layer; a second electrode is disposed in a region on an opposite edge of the elongated base layer; and a third is disposed in a region of the elongated base layer that between the first electrode and the second electrode. Optionally, the working electrode, the counter electrode and the reference electrode are positionally distributed on conductive layer in a configuration arranged so that the working electrode is disposed in a region on a first edge of the elongated base layer; the counter electrode is disposed in a region on an opposite edge of the elongated base layer; and the reference electrode is disposed in a region of the elongated base layer that between the working electrode and the counter electrode. In certain embodiments of the invention, an edge or center of a reference electrode is lined up with an edge or center of the working or counter electrode. In other embodiments of the invention, an edge or center of a reference electrode is offset with an edge or center of the working or counter electrode.

A typical embodiment of the invention comprises a multiple-conductor electrical lead, comprising: a central core element; and one or more electrical conduits (e.g. in the form of a ribbon cable) coiled around the central core along a length of the central core. Typically, the electrical conduit is disposed in a ribbon cable having one or more (typically a plurality) separate electrical conduits coupled together along their lengths in series and electrically insulated from one another with an insulating material. In a specific illustrative embodiment, the multiple-conductor electrical lead comprises 1, 2, 3, 4, 5, 6 or more electrical conduits coiled around a central strand or fibril, one that is typically made from a material selected to provide flexibility and/or tensile strength to the lead (e.g. a polyester strand). This internal strand or core element can comprise a single fiber or filament, or alternatively a plurality of one or more fibers or filaments that are intertwined, so that this core element forms a flexible central strand around which conductive elements (e.g. ribbon cables) are wrapped in a space saving configuration. The central core element can be made from one or more compositions in order to control the material properties of the multiple-conductor electrical lead. For example, in some embodiments of the invention, the core can comprises an elastic fiber such as a fiber made from a polymeric composition. A wide variety of polymeric compounds are known in the art, for example synthetic rubber, Bakelite, neoprene, nylon, PVC, polystyrene, polyethylene, polypropylene, polyacrylonitrile, thermoplastic polyamide, PVB, silicone, and the like. In some embodiments, the core comprises a metallic composition, for example a stainless steel composition or a nickel titanium composition having shape memory or super elastic properties. In one exemplary embodiment of the invention that comprises a shape memory alloy (e.g. Nitinol®), the shape memory alloy is designed to favor a space saving coiled configuration. In some embodiments, the central core element comprises both a polymeric composition and a metallic composition.

While a variety conductive materials can be used as electrical conduits in embodiments of the invention, the flexible stability of the multiple-conductor electrical leads disclosed herein allows for greater use of noble metal conductors (e.g. platinum, gold, silver iridium and copper). Consequently, in certain embodiments of the invention, the multi-conductor lead comprises a noble metal conductor and is designed so that the conductor is coupled to and supported by the flexible central core (e.g. one made from a polymeric material such as a polyester) in a manner that decreases problems associated with the inflexibility and/or fragility of certain noble metal conductor compositions.

A feature of the multi-conductor electrical lead design disclosed herein is that it is scalable, and it not for example limited to a specific number of electrical conduits and/or ribbon cables. For example, embodiments of the invention can comprise 1, 2, 3, 4 or more ribbon cables coiled around a flexible central strand, with this first coiled structure further functioning as a core element that has 1, 2, 3, 4 or more further ribbon cables wrapped around it. One such embodiment comprises a 4 conductor ribbon cable wrapped around a flexible polyester cord to form a first coiled structure, with two 6 conductor ribbon cables being further wrapped around this first coiled structure. In certain embodiments of the invention, a secondary coiled structure can function as a tertiary central core element around which further ribbon cables are wrapped. In this manner, the configuration of the leads disclosed herein optimizes the use of space.

Embodiments of the invention include methods for producing a multiple-conductor electrical lead. In one such embodiment, the method comprises supporting a central core; and wrapping at least one ribbon cable continuously around the supported central core along a length of the central core such that the at least one ribbon cable is coiled around the central core, the at least one ribbon cable comprising a plurality of separate electrical conductors coupled together along their lengths in series and electrically insulated from one another with an insulating material. Typically in such methods, the at least one ribbon cable is coiled around the central core having a broad side towards the central core. In certain methods, wrapping the at least one ribbon cable comprises wrapping a first cable layer around the central core and wrapping one or more additional cable layers around the first cable layer over the first cable layer. Optionally, the at least one ribbon cable is a pair of ribbon cables wrapped around the central core together and disposed adjacent to each other along their edges coiled around the central core.

In the methods for producing the coiled multi-conductor electrical lead configurations disclosed herein, factors such as cable wrapping pitch and wrapping tension can be controlled in a manner that stabilizes the lead components and/or optimizes the space saving architecture of the lead. For example, in certain embodiments of the invention, the wrapping pitch of a cable around a central core is set so as to abut an existing ribbon cable coil while avoiding an overlap with this existing coil. Alternatively, the wrapping pitch of a cable around a central core is set so that with each consecutive wrapping, the cable is wrapped around the core so as to overlap with an existing cable coil by a ¼, ½ or ¾ width. In addition, it is possible to optimize the inner pressure between the adjacent cables throughout the entire lead by adjusting the cable width, the amount of overlap and the cable wrapping tension. The choice and range of these parameters can be used to properly balance lead conductivity, flexibility and stability, for example by adjusting the diameter of the core elements, the number of electrical cables, their spacing and the compositions of these elements. In addition, certain embodiments of the invention can include one or more layers of a material (e.g. an adhesive material or the like, an electrically non-conducting shielding material or the like etc.) incorporated into and/or disposed around the multi-conductor electrical leads, in order to, for example, adhere, support and/or electrically shield one or more elements of the multi-conductor electrical lead (see, e.g., U.S. Patent Nos. 4,654,476 and 7,417,191, the contents of which are incorporated herein by reference).

One embodiment of the invention is a system for monitoring an analyte in a patient, the system comprising a sensor having: a base element adapted to secure the apparatus to the patient; a first piercing member coupled to and extending from the base element; and a first electrochemical sensor operatively coupled to the first piercing member and comprising a first electrochemical sensor electrode for determining at least one physiological characteristic of the patient at a first electrochemical sensor placement site. In some embodiments of such systems, a second piercing member is coupled to and extends from the base element and comprises for example: (1) a second electrochemical sensor operatively coupled to the second piercing member and comprising a second electrochemical sensor electrode for determining at least one physiological characteristic of the patient at a second electrochemical sensor placement site; or (2) a cannula or the like adapted to deliver a fluid medication (e.g. insulin) a the user.

As noted above, in typical embodiments of the invention, the sensor used with a multiple-conductor electrical lead as disclosed herein is operatively coupled to further elements (e.g. electronic components) such as elements designed to transmit and/or receive a signal, monitors, processors and the like as well as devices that can use sensor data to modulate a patient's physiology such as medication (e.g. insulin) infusion pumps. For example, in some embodiments of the invention, the sensor is operatively coupled to a sensor input capable of receiving a signal from the sensor that is based on a sensed physiological characteristic value in the mammal; and a processor coupled to the sensor input, wherein the processor is capable of characterizing one or more signals received from the sensor. A wide variety of sensor configurations as disclosed herein can be used in such systems. Optionally, for example, the sensor comprises three working electrodes, one counter electrode and one reference electrode. In certain embodiments, at least one working electrode is coated with an analyte sensing layer comprising glucose oxidase and at least one working electrode is not coated with an analyte sensing layer comprising glucose oxidase.

Embodiments of the multi-conductor lead design are particularly useful in device applications that involve DC potential, for example DC biased sensing applications. In typical applications, the medical device used with this lead (e.g. an amperometric glucose sensor) is operatively coupled to a constellation of elements that comprise a flex-circuit (e.g. electrodes, electrical conduits, contact pads and the like). For example sensor flex-circuit designs can be used in embodiments of the invention in order to optimize medical device layouts and connection schemes. One such illustrative sensor flex-circuit embodiment has 2 columns of contact pads on the left with the electrodes on the right, wherein the close proximity of the pads in this design allows for ease of connection to multi-conductor cable as well as a compact design. FIGS. 5A-5D provide schematics of illustrative sensor flex layouts for use with embodiments for the invention.

The multi-conductor lead design configurations disclosed herein provide a compact bundle of electrical conduits that takes up relatively little space. The space saving configuration provides a number of desirable properties and, for example can reduce the amount of trauma that occurs at an *in vivo* implantation site (e.g. the implantation site of an electrochemical glucose sensor of the type used in the management of diabetes). Consequently, embodiments of the invention include methods for inhibiting the likelihood of trauma at the site where a medical device is implanted *in vivo*, the method comprising supplying power to the implanted to device using an embodiment of the multi-conductor lead design configurations disclosed herein. In illustrative embodiments of this method, the implanted device in an amperometric glucose sensor. Moreover, in certain embodiments, multi-conductor lead embodiments of the invention allow an implanted device (e.g. a glucose sensor) to operate at a location that is distal (farther away) from a site of implantation than is typically possible using conventional electronic leads. Consequently, embodiments of the invention include methods for selecting a location where a medical device is implanted *in vivo*, the method comprising selecting a distal site as one both compatible with a multi-conductor lead design configuration as well as being optimized for patient comfort and then supplying power to the implanted to device using an embodiment of the multi-conductor lead design configurations disclosed herein. Certain embodiments of the invention include methods of using a specific multi-conductor lead and device (e.g. sensor) element and/or a specific constellation of sensor elements to produce and/or facilitate one or more properties of the device (e.g. to enhance its biocompatibility profile). Typical embodiments of the invention are comprised of biocompatible materials and/or have structural elements and organizations of elements designed for use in a medical device system that includes elements implanted within a mammal.

Another illustrative methodological embodiment of the invention is a method of sensing an analyte within the body of a mammal, the method comprising implanting an analyte sensor that is part of a systems that comprises a multi-conductor lead as disclosed herein in to the mammal and then sensing one or more electrical fluctuations such as alteration in current at the working electrode and correlating the alteration in current with the presence of the analyte, so that the analyte is sensed. In one such method, the analyte sensor apparatus senses glucose in the mammal. In an alternative method, the analyte sensor apparatus senses lactate, potassium, calcium, oxygen, PH, and/or any physiologically relevant analyte in the mammal. Optionally such methods utilize an electrical signal whose polarity is fixed and whose amplitude remains constant with respect to time

In some sensor system embodiments of the invention that comprise a multi-conductor lead, a processor is capable of comparing a first signal received from a working electrode in response to a first working potential with a second signal received from a working electrode in response to a second working potential, wherein the comparison of the first and second signals at the first and second working potentials can be used to, for example, identify a signal generated by an interfering compound. In one such embodiment of the invention, one working electrode is coated with glucose oxidase and another is not, and the interfering compound is acetaminophen, ascorbic acid, bilirubin, cholesterol, creatinine, dopamine, ephedrine, ibuprofen, L-dopa, methyldopa, salicylate, tetracycline, tolazamide, tolbutamide, triglycerides or uric acid. Optionally, a pulsed and/or varied voltage is used to obtain a signal from a working electrode. Typically, at least one working potential is 280, 535 or 635 millivolts. Related embodiments of the invention include methods for identifying and/or characterizing one or more signals generated by an interfering compound in various sensor embodiments of the invention (e.g. by comparing the signal from an electrode coated with an analyte sensing compound with a comparative electrode not coated with an analyte sensing compound). Optionally, such methods use a pulsed and/or varied working potential to observe a signal at an electrode.

In one glucose sensor system embodiment that comprises a multi-conductor lead as disclosed herein, a processor compares a first signal received from a working electrode coated with glucose oxidase in response to a first working potential with a second signal received from a working electrode coated with glucose oxidase in response to a second working potential, wherein the comparison of the first and second signals at the first and second working potentials is used to characterize a blood glucose concentration within at least one discreet concentration range. In such embodiments of the invention, the comparison of the first and second signals at the first and second working potentials can be used to characterize a blood glucose concentration within a concentration range below 70 mg/dL or above 125 mg/dL. Related embodiments of the invention include methods for identifying and/or characterizing a specific analyte concentration or range of analyte concentrations using the various sensor embodiments of the invention (e.g. by comparing the analyte signal from one or more electrodes at different working potentials, wherein the different working potentials are selected for their ability to characterize a specific analyte concentration and/or range of analyte concentrations).

In another glucose sensor system embodiment of the invention that comprises a multi-conductor lead, the processor is capable of characterizing a plurality of signals received from the sensor by for example comparing a first signal received from a working electrode coated with glucose oxidase with a second signal received from a working electrode not coated with glucose oxidase so as to obtain information on a background signal that is not based on a sensed physiological characteristic value in the mammal. In such embodiments of the invention, the processor can be capable of characterizing a plurality of signals received from the sensor by comparing a first signal received from a working electrode coated with glucose oxidase with a second signal received from a working electrode not coated with glucose oxidase so as to obtain information on a signal generated by an interfering compound. In a related embodiment of the invention, two working electrodes are coated with glucose oxidase and the processor is capable of obtaining information on glucose concentrations in the mammal by comparing the signals received from the two working electrodes coated with glucose oxidase.

Sensor system embodiments of the invention that comprise a multi-conductor lead can use voltage switching not only in the detection of interfering species and/or specific analyte concentrations but also to facilitate the hydration and/or initialization of various sensors. In particular, the time for initialization ("run in") differs for different sensors and can take hours. Such embodiments of the invention include a sensor initialization scheme involving high frequency initialization (e.g. switching of voltage potentials). In one illustrative embodiment, a triple initialization profile is used where the voltage of the sensor is switched between a first potential such as 280, 535 or 635 millivolts and a second potential such as 1.070 millivolts over a period of 1, 5, 10 or 15 minutes. Certain voltage switching embodiments of the invention further incorporate voltage pulsing in the measurement of an analyte. The number of pulses used in such embodiments of the invention is typically at least 2 and can be 3, 4, 5, 6, 7, 8, 9, 10, 15, 20 or more. Pulses can be for a predetermined period of time, for example 1, 3, 5, 7, 10, 15, 30, 45, 60, 90 or 120 seconds. One illustrative example of this comprises 6 pulses, each 1, 2, 3, 4, 5 or 6 seconds long. By using such embodiments of the invention, the sensor run-in is greatly accelerated, a factor which optimizes a user's introduction and activation of the sensor.

Some sensor system embodiments of the invention that comprise a multi-conductor lead can use feedback from sensor signals to provide information to a user as to start up status and/or instructions as to when to start sensing. For example, in the embodiments disclosed herein, one can use the value of an open circuit potential as a way to measure if a sensor is completely hydrated. In particular, mechanistically, in any potentiostat, one observes the difference between a working electrode and a reference electrode. This potential changes depending upon the hydration of the sensor. In the sensor is not hydrated, the circuit potential is very high (e.g. 400-500 millivolts). This circuit potential then changes as the sensor becomes hydrated. One illustrative embodiment of the invention comprises a method of detecting whether a sensor is sufficiently hydrated for analyte detection, comprising calculating an open circuit potential value (e.g. an impedance value) between at least two electrodes of the sensor; and comparing the impedance value against a threshold to determine if the sensor sufficiently hydrated for analyte detection. Yet another embodiment of the invention is an analyte sensor apparatus that includes a processor that detects whether a sensor is sufficiently hydrated for analyte detection comprising calculating an impedance value; and comparing the impedance value against a threshold to determine if the sensor is sufficiently hydrated for analyte detection. Certain embodiments of the invention are designed include an alarm signal (e.g. a indicator light, a bell, whistle or the like) that is triggered under certain specific circumstances, for example when the sensor system registers an impedance value indicating that it sufficiently hydrated for analyte detection (and in this way informs a user of the status of the sensor).

In typical sensor system embodiments of the invention, a processor determines a dynamic behavior of the physiological characteristic value and provides an observable indicator based upon the dynamic behavior of the physiological characteristic value so determined. The process of analyzing the received signal and determining a dynamic behavior typically includes repeatedly measuring the physiological characteristic value to obtain a series of physiological characteristic values in order to, for example, incorporate comparative redundancies into a sensor apparatus in a manner designed to provide confirmatory information on sensor function, analyte concentration measurements, the presence of interferents and the like. Embodiments of the invention include device systems operatively coupled to a multi-conductor lead which display data from measurements of a sensed physiological characteristic (e.g. blood glucose concentrations) in a manner and format tailored to allow a user of the device to easily monitor and, if necessary, modulate the physiological status of that characteristic (e.g. modulation of blood glucose concentrations via insulin administration). An illustrative embodiment of the invention is a device comprising a multi-conductor lead, a sensor input capable of receiving a signal from a sensor, the signal being based on a sensed physiological characteristic value of a user; a memory for storing a plurality of measurements of the sensed physiological characteristic value of the user from the received signal from the sensor; and a display for presenting a text and/or graphical representation of the plurality of measurements of the sensed physiological characteristic value (e.g. text, a line graph or the like, a bar graph or the like, a grid pattern or the like or a combination thereof). Typically, the graphical representation displays real time measurements of the sensed physiological characteristic value. Such device systems can be used in a variety of contexts, for example in combination with other medical apparatuses. In some embodiments of the invention, the device system is used in combination with at least one other medical element (e.g. a medication infusion pump, a syringe, needle, cannula or the like).

Another illustrative embodiment comprises a multi-conductor lead coupled to a glucose sensor, a transmitter and pump receiver and a glucose meter. In this system, radio signals from the transmitter can be sent to the pump receiver at a defined time period, (e.g. every 5 minutes) to provide providing real-time sensor glucose (SG) values. Values/graphs are displayed on a monitor of the pump receiver so that a user can self monitor blood glucose and deliver insulin using their own insulin pump. Typically an embodiment of device disclosed herein communicates with a second medical device via a wired or wireless connection. Wireless communication can include for example the reception of emitted radiation signals as occurs with the transmission of signals via RF telemetry, infrared transmissions, optical transmission, sonic and ultrasonic transmissions and the like. Optionally, the device is operatively coupled a medication infusion pump (e.g. an insulin pump). Typically in such devices, the physiological characteristic values includes a plurality of measurements of blood glucose.

FIG. 3 provides a perspective view of one generalized embodiment of subcutaneous sensor insertion system and a block diagram of a sensor electronics device system adaptable with multi-conductor lead embodiment of the invention. Additional elements typically used with such sensor system embodiments are disclosed for example in U.S. Patent Application No. 20070163894, the contents of which are incorporated by reference. FIG. 3 provides a perspective view of a telemetered characteristic monitor system 1, including a subcutaneous sensor set 10 provided for subcutaneous placement of an active portion of a flexible sensor 12, or the like, at a selected site in the body of a user. The subcutaneous or percutaneous portion of the sensor set 10 includes a hollow, slotted insertion needle 14 having a sharpened tip 44, and a cannula 16. Inside the cannula 16 is a sensing portion 18 of the sensor 12 to expose one or more sensor electrodes 20 to the user's bodily fluids through a window 22 formed in the cannula 16. The sensing portion 18 is joined to a connection portion 24 that terminates in conductive contact pads, or the like, which are also exposed through one of the insulative layers. The connection portion 24 and the contact pads are generally adapted for a direct wired electrical connection to a suitable monitor 200 coupled to a display 314 for monitoring a user's condition in response to signals derived from the sensor electrodes 20. The connection portion 24 may be conveniently connected electrically to the monitor 200 or a characteristic monitor transmitter 400 by a connector block 28 (or the like) as shown and described in U.S. Pat. No. 5,482,473, entitled FLEX CIRCUIT CONNECTOR, which is incorporated by reference.

As shown in FIG. 3, in accordance with embodiments of the present invention, subcutaneous sensor set 10 may be configured or formed to work with either a wired or a wireless characteristic monitor system. The proximal part of the sensor 12 is mounted in a mounting base 30 adapted for placement onto the skin of a user. The mounting base 30 can be a pad having an underside surface coated with a suitable pressure sensitive adhesive layer 32, with a peel-off paper strip 34 normally provided to cover and protect the adhesive layer 32, until the sensor set 10 is ready for use. The mounting base 30 includes upper and lower layers 36 and 38, with the connection portion 24 of the flexible sensor 12 being sandwiched between the layers 36 and 38. The connection portion 24 has a forward section joined to the active sensing portion 18 of the sensor 12, which is folded angularly to extend downwardly through a bore 40 formed in the lower base layer 38. Optionally, the adhesive layer 32 (or another portion of the apparatus in contact with *in vivo* tissue) includes an anti-inflammatory agent to reduce an inflammatory response and/or anti-bacterial agent to reduce the chance of infection. The insertion needle 14 is adapted for slide-fit reception through a needle port 42 formed in the upper base layer 36 and further through the lower bore 40 in the lower base layer 38. After insertion, the insertion needle 14 is withdrawn to leave the cannula 16 with the sensing portion 18 and the sensor electrodes 20 in place at the selected insertion site. In this embodiment, the telemetered characteristic monitor transmitter 400 is coupled to a sensor set 10 by a cable 202 (e.g. one comprising a multi-conductor lead as disclosed herein) through a connector 204 that is electrically coupled to the connector block 28 of the connector portion 24 of the sensor set 10.

In the embodiment shown in FIG. 3, the telemetered characteristic monitor 400 includes a housing 206 that supports a printed circuit board 208, batteries 210, antenna 212, and the cable 202 with the connector 204. In some embodiments, the housing 206 is formed from an upper case 214 and a lower case 216 that are sealed with an ultrasonic weld to form a waterproof (or resistant) seal to permit cleaning by immersion (or swabbing) with water, cleaners, alcohol or the like. In some embodiments, the upper and lower case 214 and 216 are formed from a medical grade plastic. However, in alternative embodiments, the upper case 214 and lower case 216 may be connected together by other methods, such as snap fits, sealing rings, RTV (silicone sealant) and bonded together, or the like, or formed from other materials, such as metal, composites, ceramics, or the like. In other embodiments, the separate case can be eliminated and the assembly is simply potted in epoxy or other moldable materials that is compatible with the electronics and reasonably moisture resistant. As shown, the lower case 216 may have an underside surface coated with a suitable pressure sensitive adhesive layer 118, with a peel-off paper strip 120 normally provided to cover and protect the adhesive layer 118, until the sensor set telemetered characteristic monitor transmitter 400 is ready for use.

In the illustrative embodiment shown in FIG. 3, the subcutaneous sensor set 10 facilitates accurate placement of a flexible thin film electrochemical sensor 12 of the type used for monitoring specific blood parameters representative of a user's condition. The sensor 12 monitors glucose levels in the body, and may be used in conjunction with automated or semi-automated medication infusion pumps of the external or implantable type as described in U.S. Pat. Nos. 4,562,751; 4,678,408; 4,685,903 or 4,573,994, to control delivery of insulin to a diabetic patient. In the illustrative embodiment shown in FIG. 3, the sensor electrodes 10 may be used in a variety of sensing applications and may be configured in a variety of ways. For example, the sensor electrodes 10 may be used in physiological parameter sensing applications in which some type of biomolecule is used as a catalytic agent. For example, the sensor electrodes 10 may be used in a glucose and oxygen sensor having a glucose oxidase enzyme catalyzing a reaction with the sensor electrodes 20. The sensor electrodes 10, along with a biomolecule or some other catalytic agent, may be placed in a human body in a vascular or non-vascular environment. For example, the sensor electrodes 20 and biomolecule may be placed in a vein and be subjected to a blood stream, or may be placed in a subcutaneous or peritoneal region of the human body.

In the embodiment of the invention shown in FIG. 3, the monitor of sensor signals 200 may also be referred to as a sensor electronics device 200. The monitor 200 may include a power source, a sensor interface, processing electronics (i.e. a processor), and data formatting electronics. The monitor 200 may be coupled to the sensor set 10 by a cable 202 through a connector that is electrically coupled to the connector block 28 of the connection portion 24. In an alternative embodiment, the cable may be omitted. In this embodiment of the invention, the monitor 200 may include an appropriate connector for direct connection to the connection portion 204 of the sensor set 10. The sensor set 10 may be modified to have the connector portion 204 positioned at a different location, e.g., on top of the sensor set to facilitate placement of the monitor 200 over the sensor set.

While the analyte sensor and sensor systems disclosed herein are typically designed to be implantable within the body of a mammal, the inventions disclosed herein are not limited to any particular environment and can instead be used in a wide variety of contexts, for example for the analysis of most *in vivo* and in vitro liquid samples including biological fluids such as interstitial fluids, whole-blood, lymph, plasma, serum, saliva, urine, stool, perspiration, mucus, tears, cerebrospinal fluid, nasal secretion, cervical or vaginal secretion, semen, pleural fluid, amniotic fluid, peritoneal fluid, middle ear fluid, joint fluid, gastric aspirate or the like. In addition, solid or desiccated samples may be dissolved in an appropriate solvent to provide a liquid mixture suitable for analysis.

In some amperometric sensor embodiments that use a multi-conductor lead, distributed electrodes of a sensor are organized/disposed within a flex-circuit assembly (i.e. a circuitry assembly that utilizes flexible rather than rigid materials). Such flex-circuit assembly embodiments provide an interconnected assembly of elements (e.g. electrodes, electrical conduits, contact pads and the like) configured to facilitate wearer comfort (for example by reducing pad stiffness and wearer discomfort) as well as parameter measurement performance. FIGS. 5A-5D show sensor flex layouts that can be used in embodiments of the invention in order to optimize the dual sensor layout and connection scheme. The embodiment shown in FIG. 5A has two columns of contact pads on the left with the electrodes on the right. The close proximity of the pads in this design allows for ease of connection to cable as well as a compact design. The embodiment shown in FIG. 5B has the two columns of contact pads at the center in between both sensor electrodes. Embodiments having the electrodes on the opposite side maximizes sensor separation while keeping both contact pads together. The embodiment shown in FIG. 5C has a single column of contact pads allowing for a different connection scheme with more width space than the design shown in FIG 5A. The embodiment shown in FIG. 5D benefits from a staggered element layout which allowing it to be compact yet still retain spacing between electrodes sets. In some embodiments of the invention, the contact pads can be arranged near the edges of the flexible sensor substrate, with leads on the substrate connecting the sensors to the contact pads, for example to prevent the contact pads from being contaminated with the materials being tested. Embodiment can include printed circuit boards having a plurality of board contact pads arranged in the same configuration as the sensor contact pads in the sensor array. Connectors, such as conducting elastomers, stick probes, cantilever probes, conducting adhesives, wafer-to-board bonding techniques, or other contact devices, can couple the sensors with the printed circuit board by creating contacts between the sensor contact pads and the board contact pads, preferably the contacts are reversible and non-permanent. Certain flex assemblies than can be modified and or adapted for use with embodiments of the invention are disclosed for example in U.S. Patent Nos. 7,340,287, 7,377,794 and 6,930,494 the contents of which are incorporated by reference.

Embodiments of the invention that can be used with the multi-conductor leads disclosed herein include sensors and sensor systems having configurations of elements and/or architectures that optimize aspects of sensor function. For example, certain embodiments of the invention are constructed to include multiple and/or redundant elements such as multiple sets of sensors and/or sensor system elements such as multiple piercing members (e.g. needles) and/or a cannulas organized on an insertion apparatus for use at a patient's *in vivo* insertion site. One embodiment of the invention is the dual piercing member or "fang" sensor system. This embodiment of the invention is a sensor apparatus for monitoring a body characteristic of the patient, the apparatus comprising a base element adapted to secure the apparatus to the patient, a first piercing member that is coupled to and extending from the base element, wherein the first piercing member is operatively coupled to (e.g. to provide structural support and/or enclose) at least one first electrochemical sensor having at least one electrode for determining at least one body characteristic of the patient at a first sensor placement site, as well as a second piercing member that is coupled to and extending from the base element and operatively coupled to at least one second electrochemical sensor having at least one electrode for determining at least one body characteristic of the patient at a second sensor placement site. In some embodiments of the invention, such sensor systems are used in a hospital setting such as in an intensive care unit (e.g. to measure blood glucose concentrations in the interstitial fluid or blood of a diabetic patient). In other embodiments of the invention, the apparatus is used in an ambulatory context, for example by a diabetic in the daily monitoring of blood glucose.

Typical sensor system embodiments of the invention include a sensor, a multi-conductor lead and a processor which compiles and processes signals produced by the sensors and, for example, then provides a physiological characteristic reading that is based upon the signals. In one illustrative embodiment, the processor uses an algorithm to provide computational comparisons between a plurality of sensors having elements coated with an oxidoreductase such as glucose oxidase in order to, for example provide a comparative assessment of a physiological characteristic such as blood glucose at the different sites in which the sensors are inserted. In another illustrative embodiment, the processor includes an algorithm which provides a computational comparison between a plurality of sensors including at least one sensor having elements coated with an oxidoreductase such as glucose oxidase and at least one sensor not coated with glucose oxidase (e.g. which functions to identify background or interfering signals unrelated to blood glucose) in order to, for example subtract signals unrelated to blood glucose and in this way provide optimized sensor outputs. Certain embodiments of the invention include apparatuses capable of multiplexing the signals received from the plurality of sensors, e.g. by weaving multiple sensor signals onto a single channel or communications line. In such multiplexing embodiments of the invention, segments of information from each signal can be interleaved and separated by time, frequency, or space in order to obtain a comparative and comprehensive reading of all sensor outputs. Certain multiplexing embodiments of the invention include a processor which uses an algorithm to provide computational comparisons between signals received from the plurality of sensors (e.g. to provide a mean, average or normalized value for the sensor signals).

Sensor systems comprising the multi-conductor leads disclosed herein can be used to sense analytes of interest in one or more physiological environments. In certain embodiments for example, the sensor can be in direct contact with interstitial fluids as typically occurs with subcutaneous sensors. The sensor systems of the present invention may also be part of a skin surface system where interstitial glucose is extracted through the skin and brought into contact with the sensor (see, e.g. U.S. Patent Nos. 6,155,992 and 6,706,159 which are incorporated herein by reference). In other embodiments, the sensor can be in contact with blood as typically occurs for example with intravenous sensors. The multi-conductor leads disclosed herein allow sensor systems to be adapted for use in a variety of contexts. In certain embodiments for example, the sensor system can be designed for use in mobile contexts, such as those employed by ambulatory users (e.g. a diabetic user performing daily activities). Alternatively, the sensor system can be designed for use in stationary contexts such as those adapted for use in clinical settings. Such sensor system embodiments include, for example, those used to monitor one or more analytes present in one or more physiological environments in a hospitalized patient (e.g. a patient confined to a hospital bed in situations such as those described in WO 2008042625).

The multi-conductor leads disclosed herein be incorporated in to a wide variety of medical systems known in the art. Sensor systems coupled to such leads can be used, for example, in a closed loop infusion systems designed to control the rate that medication is infused into the body of a user. Such a closed loop infusion system can include a sensor and an associated meter which generates an input to a controller which in turn operates a delivery system (e.g. one that calculates a dose to be delivered by a medication infusion pump). In such contexts, the meter associated with the sensor may also transmit commands to, and be used to remotely control, the delivery system. Typically, the sensor is a subcutaneous sensor in contact with interstitial fluid to monitor the glucose concentration in the body of the user, and the liquid infused by the delivery system into the body of the user includes insulin. Illustrative systems are disclosed for example in U. S. Patent Nos. 6,558,351 and 6,551,276; PCT Application Nos. US99/21703 and US99/22993; as well as WO 2004/008956 and WO 2004/009161, all of which are incorporated herein by reference.

In another embodiment of the invention, a kit and/or device set (e.g. a sensor useful for the sensing an analyte as is described above) is provided. The kit and/or device set typically comprises a container, a label, a multi-conductor lead and a device as described herein (e.g. an amperometric glucose sensor). Suitable containers include, for example, an easy to open package made from a material such as a metal foil, bottles, vials, syringes, and test tubes. The containers may be formed from a variety of materials such as metals (e.g. foils) paper products, glass or plastic. The label on, or associated with, the container indicates the preferred device use. The kit and/or device set may further include other materials desirable from a commercial and user standpoint, including elements or devices designed to facilitate the introduction of the device into an *in vivo* environment, other buffers, diluents, filters, needles, syringes, and package inserts with instructions for use.

An exemplary kit comprises a container and, within the container, multi-conductor lead for use with an analyte sensor apparatus, the sensor apparatus comprising a base layer; a conductive layer disposed upon the base layer; wherein the conductive layer includes a working electrode; an analyte sensing layer disposed on the conductive layer; wherein the analyte sensing layer detectably alters the electrical current at the working electrode in the conductive layer in the presence of an analyte; and an analyte modulating layer disposed on the analyte sensing layer, wherein the analyte modulating layer modulates the diffusion of the analyte therethrough.

### TYPICAL SENSOR ACHITECTURES FOUND IN OF EMBODIMENTS OF THE INVENTION

A variety of sensors can be operatively coupled to the multi-conductor leads disclosed herein. FIG. 2 illustrates a cross-section of a typical sensor embodiment 100 for use with the multi-conductor lead embodiments of the present invention. This sensor embodiment is formed from a plurality of components that are typically in the form of layers of various conductive and non-conductive constituents disposed on each other according to art accepted methods and/or the specific methods of the invention disclosed herein. The components of the sensor are typically characterized herein as layers because, for example, it allows for a facile characterization of the sensor structure shown in FIG. 2. Artisans will understand however, that in certain embodiments of the invention, the sensor constituents are combined such that multiple constituents form one or more heterogeneous layers. In this context, those of skill in the art understand that the ordering of the layered constituents can be altered in various embodiments of the invention.

The embodiment shown in FIG. 2 includes a base layer 102 to support the sensor 100. The base layer 102 can be made of a material such as a metal and/or a ceramic and/or a polymeric substrate, which may be self-supporting or further supported by another material as is known in the art. Embodiments of the invention include a conductive layer 104 which is disposed on and/or combined with the base layer 102. Typically the conductive layer 104 comprises one or more electrodes. An operating sensor 100 typically includes a plurality of electrodes such as a working electrode, a counter electrode and a reference electrode. Other embodiments may also include a plurality of working and/or counter and/or reference electrodes and/or one or more electrodes that performs multiple functions, for example one that functions as both as a reference and a counter electrode.

As discussed in detail below, the base layer 102 and/or conductive layer 104 can be generated using many known techniques and materials. In certain embodiments of the invention, the electrical circuit of the sensor is defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating cover layer 106 such as a polymer coating can be disposed on portions of the sensor 100. Acceptable polymer coatings for use as the insulating protective cover layer 106 can include, but are not limited to, non-toxic biocompatible polymers such as silicone compounds, polyimides, biocompatible solder masks, epoxy acrylate copolymers, or the like. In the sensors of the present invention, one or more exposed regions or apertures 108 can be made through the cover layer 106 to open the conductive layer 104 to the external environment and to, for example, allow an analyte such as glucose to permeate the layers of the sensor and be sensed by the sensing elements. Apertures 108 can be formed by a number of techniques, including laser ablation, tape masking, chemical milling or etching or photolithographic development or the like. In certain embodiments of the invention, during manufacture, a secondary photoresist can also be applied to the protective layer 106 to define the regions of the protective layer to be removed to form the aperture(s) 108. The exposed electrodes and/or contact pads can also undergo secondary processing (e.g. through the apertures 108), such as additional plating processing, to prepare the surfaces and/or strengthen the conductive regions.

In the sensor configuration shown in FIG. 2, an analyte sensing layer 110 (which is typically a sensor chemistry layer, meaning that materials in this layer undergo a chemical reaction to produce a signal that can be sensed by the conductive layer) is disposed on one or more of the exposed electrodes of the conductive layer 104. Typically, the analyte sensing layer 110 is an enzyme layer. Most typically, the analyte sensing layer 110 comprises an enzyme capable of producing and/or utilizing oxygen and/or hydrogen peroxide, for example the enzyme glucose oxidase. Optionally the enzyme in the analyte sensing layer is combined with a second carrier protein such as human serum albumin, bovine serum albumin or the like. In an illustrative embodiment, an oxidoreductase enzyme such as glucose oxidase in the analyte sensing layer 110 reacts with glucose to produce hydrogen peroxide, a compound which then modulates a current at an electrode. As this modulation of current depends on the concentration of hydrogen peroxide, and the concentration of hydrogen peroxide correlates to the concentration of glucose, the concentration of glucose can be determined by monitoring this modulation in the current. In a specific embodiment of the invention, the hydrogen peroxide is oxidized at a working electrode which is an anode (also termed herein the anodic working electrode), with the resulting current being proportional to the hydrogen peroxide concentration. Such modulations in the current caused by changing hydrogen peroxide concentrations can by monitored by any one of a variety of sensor detector apparatuses such as a universal sensor amperometric biosensor detector or one of the other variety of similar devices known in the art such as glucose monitoring devices produced by Medtronic MiniMed.

In embodiments of the invention, the analyte sensing layer 110 can be applied over portions of the conductive layer or over the entire region of the conductive layer. Typically the analyte sensing layer 110 is disposed on the working electrode which can be the anode or the cathode. Optionally, the analyte sensing layer 110 is also disposed on a counter and/or reference electrode. While the analyte sensing layer 110 can be up to about 1000 microns (µm) in thickness, typically the analyte sensing layer is relatively thin as compared to those found in sensors previously described in the art, and is for example, typically less than 1, 0.5, 0.25 or 0.1 microns in thickness. As discussed in detail below, some methods for generating a thin analyte sensing layer 110 include brushing the layer onto a substrate (e.g. the reactive surface of a platinum black electrode), as well as spin coating processes, dip and dry processes, low shear spraying processes, ink-jet printing processes, silk screen processes and the like. In certain embodiments of the invention, brushing is used to: (1) allow for a precise localization of the layer; and (2) push the layer deep into the architecture of the reactive surface of an electrode (e.g. platinum black produced by an electrodeposition process).

Typically, the analyte sensing layer 110 is coated and or disposed next to one or more additional layers. Optionally, the one or more additional layers includes a protein layer 116 disposed upon the analyte sensing layer 110. Typically, the protein layer 116 comprises a protein such as human serum albumin, bovine serum albumin or the like. Typically, the protein layer 116 comprises human serum albumin. In some embodiments of the invention, an additional layer includes an analyte modulating layer 112 that is disposed above the analyte sensing layer 110 to regulate analyte contact with the analyte sensing layer 110. For example, the analyte modulating membrane layer 112 can comprise a glucose limiting membrane, which regulates the amount of glucose that contacts an enzyme such as glucose oxidase that is present in the analyte sensing layer. Such glucose limiting membranes can be made from a wide variety of materials known to be suitable for such purposes, e.g., silicone compounds such as polydimethyl siloxanes, polyurethanes, polyurea cellulose acetates, Nafion, polyester sulfonic acids (e.g. Kodak AQ), hydrogels or any other suitable hydrophilic membranes known to those skilled in the art.

In typical embodiments of the invention, an adhesion promoter layer 114 is disposed between the analyte modulating layer 112 and the analyte sensing layer 110 as shown in FIG. 2 in order to facilitate their contact and/or adhesion. In a specific embodiment of the invention, an adhesion promoter layer 114 is disposed between the analyte modulating layer 112 and the protein layer 116 as shown in FIG. 2 in order to facilitate their contact and/or adhesion. The adhesion promoter layer 114 can be made from any one of a wide variety of materials known in the art to facilitate the bonding between such layers. Typically, the adhesion promoter layer 114 comprises a silane compound. In alternative embodiments, protein or like molecules in the analyte sensing layer 110 can be sufficiently crosslinked or otherwise prepared to allow the analyte modulating membrane layer 112 to be disposed in direct contact with the analyte sensing layer 110 in the absence of an adhesion promoter layer 114.

In certain embodiments of the invention, a sensor is designed to include additional layers such as an interference rejection layer discussed below.

### TYPICAL ANALYTE SENSOR CONSTITUENTS USED IN EMBODIMENTS OF THE INVENTION

The following disclosure provides examples of typical elements/constituents used in sensor embodiments of the invention. While these elements can be described as discreet units (e.g. layers), those of skill in the art understand that sensors can be designed to contain elements having a combination of some or all of the material properties and/or functions of the elements/constituents discussed below (e.g. an element that serves both as a supporting base constituent and/or a conductive constituent and/or a matrix for the analyte sensing constituent and which further functions as an electrode in the sensor). Those in the art understand that these thin film analyte sensors can be adapted for use in a number of sensor systems such as those described below.

### BASE CONSTITUENT

Sensors of the invention typically include a base constituent (see, e.g. element 102 in Figure 2). The term "base constituent" is used herein according to art accepted terminology and refers to the constituent in the apparatus that typically provides a supporting matrix for the plurality of constituents that are stacked on top of one another and comprise the functioning sensor. In one form, the base constituent comprises a thin film sheet of insulative (e.g. electrically insulative and/or water impermeable) material. This base constituent can be made of a wide variety of materials having desirable qualities such as dielectric properties, water impermeability and hermeticity. Some materials include metallic, and/or ceramic and/or polymeric substrates or the like.

The base constituent may be self-supporting or further supported by another material as is known in the art. In one embodiment of the sensor configuration shown in Figure 2, the base constituent 102 comprises a ceramic. Alternatively, the base constituent comprises a polymeric material such as a polyimmide. In an illustrative embodiment, the ceramic base comprises a composition that is predominantly Al₂O₃ (e.g. 96%). The use of alumina as an insulating base constituent for use with implantable devices is disclosed in U.S. Pat. Nos. 4,940,858, 4,678,868 and 6,472,122 which are incorporated herein by reference. The base constituents of the invention can further include other elements known in the art, for example hermetical vias (see, e.g. WO 03/023388). Depending upon the specific sensor design, the base constituent can be relatively thick constituent (e.g. thicker than 50, 100, 200, 300, 400, 500 or 1000 microns). Alternatively, one can utilize a nonconductive ceramic, such as alumina, in thin constituents, e.g., less than about 30 microns.

### CONDUCTIVE CONSTITUENT

The electrochemical sensors of the invention typically include a conductive constituent disposed upon the base constituent that includes at least one electrode for contacting an analyte or its byproduct (e.g. oxygen and/or hydrogen peroxide) to be assayed (see, e.g. element 104 in Figure 2). The term "conductive constituent" is used herein according to art accepted terminology and refers to electrically conductive sensor elements such as electrodes which are capable of measuring and a detectable signal and conducting this to a detection apparatus. An illustrative example of this is a conductive constituent that can measure an increase or decrease in current in response to exposure to a stimuli such as the change in the concentration of an analyte or its byproduct as compared to a reference electrode that does not experience the change in the concentration of the analyte, a coreactant (e.g. oxygen) used when the analyte interacts with a composition (e.g. the enzyme glucose oxidase) present in analyte sensing constituent 110 or a reaction product of this interaction (e.g. hydrogen peroxide). Illustrative examples of such elements include electrodes which are capable of producing variable detectable signals in the presence of variable concentrations of molecules such as hydrogen peroxide or oxygen. Typically one of these electrodes in the conductive constituent is a working electrode, which can be made from non-corroding metal or carbon. A carbon working electrode may be vitreous or graphitic and can be made from a solid or a paste. A metallic working electrode may be made from platinum group metals, including palladium or gold, or a non-corroding metallically conducting oxide, such as ruthenium dioxide. Alternatively the electrode may comprise a silver/silver chloride electrode composition. The working electrode may be a wire or a thin conducting film applied to a substrate, for example, by coating or printing. Typically, only a portion of the surface of the metallic or carbon conductor is in electrolytic contact with the analyte-containing solution. This portion is called the working surface of the electrode. The remaining surface of the electrode is typically isolated from the solution by an electrically insulating cover constituent 106. Examples of useful materials for generating this protective cover constituent 106 include polymers such as polyimides, polytetrafluoroethylene, polyhexafluoropropylene and silicones such as polysiloxanes.

In addition to the working electrode, the analyte sensors of the invention typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors of the present invention have one or more working electrodes and one or more counter, reference, and/or counter/reference electrodes. One embodiment of the sensor of the present invention has two, three or four or more working electrodes. These working electrodes in the sensor may be integrally connected or they may be kept separate.

Typically for *in vivo* use, embodiments of the present invention are implanted subcutaneously in the skin of a mammal for direct contact with the body fluids of the mammal, such as blood. Alternatively the sensors can be implanted into other regions within the body of a mammal such as in the intraperotineal space. When multiple working electrodes are used, they may be implanted together or at different positions in the body. The counter, reference, and/or counter/reference electrodes may also be implanted either proximate to the working electrode(s) or at other positions within the body of the mammal. Embodiments of the invention include sensors comprising electrodes constructed from nanostructured materials. As used herein, a "nanostructured material" is an object manufactured to have at least one dimension smaller than 100 nm. Examples include, but are not limited to, single-walled nanotubes, double-walled nanotubes, multi-walled nanotubes, bundles of nanotubes, fullerenes, cocoons, nanowires, nanofibres, onions and the like.

### INTERFERENCE REJECTION CONSTITUENT

The electrochemical sensors of the invention optionally include an interference rejection constituent disposed between the surface of the electrode and the environment to be assayed. In particular, certain sensor embodiments rely on the oxidation and/or reduction of hydrogen peroxide generated by enzymatic reactions on the surface of a working electrode at a constant potential applied. Because amperometric detection based on direct oxidation of hydrogen peroxide requires a relatively high oxidation potential, sensors employing this detection scheme may suffer interference from oxidizable species that are present in biological fluids such as ascorbic acid, uric acid and acetaminophen. In this context, the term "interference rejection constituent" is used herein according to art accepted terminology and refers to a coating or membrane in the sensor that functions to inhibit spurious signals generated by such oxidizable species which interfere with the detection of the signal generated by the analyte to be sensed. Certain interference rejection constituents function via size exclusion (e.g. by excluding interfering species of a specific size). Examples of interference rejection constituents include one or more layers or coatings of compounds such as hydrophilic polyurethanes, cellulose acetate (including cellulose acetate incorporating agents such as poly(ethylene glycol), polyethersulfones, polytetra-fluoroethylenes, the perfluoronated ionomer Nafion™, polyphenylenediamine, epoxy and the like. Illustrative discussions of such interference rejection constituents are found for example in Ward et al., Biosensors and Bioelectronics 17 (2002) 181-189 and Choi et al., Analytical Chimica Acta 461 (2002) 251-260 which are incorporated herein by reference. Other interference rejection constituents include for example those observed to limit the movement of compounds based upon a molecular weight range, for example cellulose acetate as disclosed for example in U.S. Patent No. 5,755,939, the contents of which are incorporated by reference.

### ANALYTE SENSING CONSTITUENT

The electrochemical sensors of the invention include an analyte sensing constituent disposed on the electrodes of the sensor (see, e.g. element 110 in Figure 2). The term "analyte sensing constituent" is used herein according to art accepted terminology and refers to a constituent comprising a material that is capable of recognizing or reacting with an analyte whose presence is to be detected by the analyte sensor apparatus. Typically this material in the analyte sensing constituent produces a detectable signal after interacting with the analyte to be sensed, typically via the electrodes of the conductive constituent. In this regard the analyte sensing constituent and the electrodes of the conductive constituent work in combination to produce the electrical signal that is read by an apparatus associated with the analyte sensor. Typically, the analyte sensing constituent comprises an oxidoreductase enzyme capable of reacting with and/or producing a molecule whose change in concentration can be measured by measuring the change in the current at an electrode of the conductive constituent (e.g. oxygen and/or hydrogen peroxide), for example the enzyme glucose oxidase. An enzyme capable of producing a molecule such as hydrogen peroxide can be disposed on the electrodes according to a number of processes known in the art. The analyte sensing constituent can coat all or a portion of the various electrodes of the sensor. In this context, the analyte sensing constituent may coat the electrodes to an equivalent degree. Alternatively the analyte sensing constituent may coat different electrodes to different degrees, with for example the coated surface of the working electrode being larger than the coated surface of the counter and/or reference electrode.

Typical sensor embodiments of this element of the invention utilize an enzyme (e.g. glucose oxidase) that has been combined with a second protein (e.g. albumin) in a fixed ratio (e.g. one that is typically optimized for glucose oxidase stabilizing properties) and then applied on the surface of an electrode to form a thin enzyme constituent. In a typical embodiment, the analyte sensing constituent comprises a GOx and HSA mixture. In a typical embodiment of an analyte sensing constituent having GOx, the GOx reacts with glucose present in the sensing environment (e.g. the body of a mammal) and generates hydrogen peroxide according to the reaction shown in Figure 1, wherein the hydrogen peroxide so generated is anodically detected at the working electrode in the conductive constituent.

As noted above, the enzyme and the second protein (e.g. an albumin) are typically treated to form a crosslinked matrix (e.g. by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. Patent Application Serial Number 10/335,506 and PCT publication WO 03/035891 which are incorporated herein by reference. For example, an amine cross-linking reagent, such as, but not limited to, glutaraldehyde, can be added to the protein mixture. The addition of a cross-linking reagent to the protein mixture creates a protein paste. The concentration of the cross-linking reagent to be added may vary according to the concentration of the protein mixture. While glutaraldehyde is an illustrative crosslinking reagent, other cross-linking reagents may also be used. Other suitable cross-linkers also may be used, as will be evident to those skilled in the art.

The GOx and/or carrier protein concentration may vary for different embodiments of the invention. For example, the GOx concentration may be within the range of approximately 50 mg/ml (approximately 10,000 U/ml) to approximately 700 mg/ml (approximately 150,000 U/ml). Typically the GOx concentration is about 115 mg/ml (approximately 22,000 U/ml). In such embodiments, the HSA concentration may vary between about 0.5%-30% (w/v), depending on the GOx concentration. Typically the HSA concentration is about 1-10% w/v, and most typically is about 5% w/v. In alternative embodiments of the invention, collagen or BSA or other structural proteins used in these contexts can be used instead of or in addition to HSA. Although GOx is discussed as an illustrative enzyme in the analyte sensing constituent, other proteins and/or enzymes may also be used or may be used in place of GOx, including, but not limited to glucose dehydrogenase or hexokinase, hexose oxidase, lactate oxidase, and the like. Other proteins and/or enzymes may also be used, as will be evident to those skilled in the art. Moreover, although HSA is employed in the example embodiment, other structural proteins, such as BSA, collagens or the like, could be used instead of or in addition to HSA.

As noted above, in some embodiments of the invention, the analyte sensing constituent includes a composition (e.g. glucose oxidase) capable of producing a signal (e.g. a change in oxygen and/or hydrogen peroxide concentrations) that can be sensed by the electrically conductive elements (e.g. electrodes which sense changes in oxygen and/or hydrogen peroxide concentrations). However, other useful analyte sensing constituents can be formed from any composition that is capable of producing a detectable signal that can be sensed by the electrically conductive elements after interacting with a target analyte whose presence is to be detected. In some embodiments, the composition comprises an enzyme that modulates hydrogen peroxide concentrations upon reaction with an analyte to be sensed. Alternatively, the composition comprises an enzyme that modulates oxygen concentrations upon reaction with an analyte to be sensed. In this context, a wide variety of enzymes that either use or produce hydrogen peroxide and/or oxygen in a reaction with a physiological analyte are known in the art and these enzymes can be readily incorporated into the analyte sensing constituent composition. A variety of other enzymes known in the art can produce and/or utilize compounds whose modulation can be detected by electrically conductive elements such as the electrodes that are incorporated into the sensor designs described herein. Such enzymes include for example, enzymes specifically described in Table 1, pages 15-29 and/or Table 18, pages 111-112 of Protein Immobilization: Fundamentals and Applications (Bioprocess Technology, Vol 14) by Richard F. Taylor (Editor) Publisher: Marcel Dekker; (January 7, 1991) the entire contents of which are incorporated herein by reference.

Other useful analyte sensing constituents can be formed to include antibodies whose interaction with a target analyte is capable of producing a detectable signal that can be sensed by the electrically conductive elements after interacting with the target analyte whose presence is to be detected. For example U.S. Patent No. 5,427,912 (which is incorporated herein by reference) describes an antibody-based apparatus for electrochemically determining the concentration of an analyte in a sample. In this device, a mixture is formed which includes the sample to be tested, an enzyme-acceptor polypeptide, an enzyme-donor polypeptide linked to an analyte analog (enzyme-donor polypeptide conjugate), a labeled substrate, and an antibody specific for the analyte to be measured. The analyte and the enzyme-donor polypeptide conjugate competitively bind to the antibody. When the enzyme-donor polypeptide conjugate is not bound to antibody, it will spontaneously combine with the enzyme acceptor polypeptide to form an active enzyme complex. The active enzyme then hydrolyzes the labeled substrate, resulting in the generation of an electroactive label, which can then be oxidized at the surface of an electrode. A current resulting from the oxidation of the electroactive compound can be measured and correlated to the concentration of the analyte in the sample. U.S. Patent No. 5,149,630 (which is incorporated herein by reference) describes an electrochemical specific binding assay of a ligand (e.g., antigen, hapten or antibody) wherein at least one of the components is enzyme-labelled, and which includes the step of determining the extent to which the transfer of electrons between the enzyme substrate and an electrode, associated with the substrate reaction, is perturbed by complex formation or by displacement of any ligand complex relative to unbound enzyme-labelled component. U.S. Patent No. 6,410,251 (which is incorporated herein by reference) describes an apparatus and method for detecting or assaying one constituting member in a specific binding pair; for example, the antigen in an antigen/antibody pair, by utilizing specific binding such as binding between an antigen and an antibody, together with redox reaction for detecting a label, wherein an oxygen micro-electrode with a sensing surface area is used. In addition, U.S. Patent No. 4,402,819 (which is incorporated herein by reference) describes an antibody-selective potentiometric electrode for the quantitative determination of antibodies (as the analyte) in dilute liquid serum samples employing an insoluble membrane incorporating an antigen having bonded thereto an ion carrier effecting the permeability of preselected cations therein, which permeability is a function of specific antibody concentrations in analysis, and the corresponding method of analysis. For related disclosures, see also U.S. Patent Nos. 6,703,210, 5,981,203, 5,705,399 and 4,894,253, the contents of which are incorporated herein by reference.

In addition to enzymes and antibodies, other exemplary materials for use in the analyte sensing constituents of the sensors disclosed herein include polymers that bind specific types of cells or cell components (e.g. polypeptides, carbohydrates and the like); single-strand DNA; antigens and the like. The detectable signal can be, for example, an optically detectable change, such as a color change or a visible accumulation of the desired analyte (e.g., cells). Sensing elements can also be formed from materials that are essentially non-reactive (i.e., controls). The foregoing alternative sensor elements are beneficially included, for example, in sensors for use in cell-sorting assays and assays for the presence of pathogenic organisms, such as viruses (HIV, hepatitis-C, etc.), bacteria, protozoa and the like.

Also contemplated are analyte sensors that measure an analyte that is present in the external environment and that can in itself produce a measurable change in current at an electrode. In sensors measuring such analytes, the analyte sensing constituent can be optional.

### PROTEIN CONSTITUENT

The electrochemical sensors of the invention optionally include a protein constituent disposed between the analyte sensing constituent and the analyte modulating constituent (see, e.g. element 116 in Figure 2). The term "protein constituent" is used herein according to art accepted terminology and refers to constituent containing a carrier protein or the like that is selected for compatibility with the analyte sensing constituent and/or the analyte modulating constituent. In typical embodiments, the protein constituent comprises an albumin such as human serum albumin. The HSA concentration may vary between about 0.5%-30% (w/v). Typically the HSA concentration is about 1-10% w/v, and most typically is about 5% w/v. In alternative embodiments of the invention, collagen or BSA or other structural proteins used in these contexts can be used instead of or in addition to HSA. This constituent is typically crosslinked on the analyte sensing constituent according to art accepted protocols.

### ADHESION PROMOTING CONSTITUENT

The electrochemical sensors of the invention can include one or more adhesion promoting (AP) constituents (see, e.g. element 114 in Figure 2). The term "adhesion promoting constituent" is used herein according to art accepted terminology and refers to a constituent that includes materials selected for their ability to promote adhesion between adjoining constituents in the sensor. Typically, the adhesion promoting constituent is disposed between the analyte sensing constituent and the analyte modulating constituent. Typically, the adhesion promoting constituent is disposed between the optional protein constituent and the analyte modulating constituent. The adhesion promoter constituent can be made from any one of a wide variety of materials known in the art to facilitate the bonding between such constituents and can be applied by any one of a wide variety of methods known in the art. Typically, the adhesion promoter constituent comprises a silane compound such as γ-aminopropyltrimethoxysilane.

The use of silane coupling reagents, especially those of the formula R'Si(OR)₃ in which R' is typically an aliphatic group with a terminal amine and R is a lower alkyl group, to promote adhesion is known in the art (see, e.g. U.S. Patent No. 5,212,050 which is incorporated herein by reference). For example, chemically modified electrodes in which a silane such as γ-aminopropyltriethoxysilane and glutaraldehyde were used in a step-wise process to attach and to co-crosslink bovine serum albumin (BSA) and glucose oxidase (GOx) to the electrode surface are well known in the art (see, e.g. Yao, T. Analytica Chim. Acta 1983, 148, 27-33).

In certain embodiments of the invention, the adhesion promoting constituent further comprises one or more compounds that can also be present in an adjacent constituent such as the polydimethyl siloxane (PDMS) compounds that serves to limit the diffusion of analytes such as glucose through the analyte modulating constituent. In illustrative embodiments the formulation comprises 0.5-20% PDMS, typically 5-15% PDMS, and most typically 10% PDMS. In certain embodiments of the invention, the adhesion promoting constituent is crosslinked within the layered sensor system and correspondingly includes an agent selected for its ability to crosslink a moiety present in a proximal constituent such as the analyte modulating constituent. In illustrative embodiments of the invention, the adhesion promoting constituent includes an agent selected for its ability to crosslink an amine or carboxyl moiety of a protein present in a proximal constituent such a the analyte sensing constituent and/or the protein constituent and or a siloxane moiety present in a compound disposed in a proximal layer such as the analyte modulating layer.

### ANALYTE MODULATING CONSTITUENT

The electrochemical sensors of the invention include an analyte modulating constituent disposed on the sensor (see, e.g. element 112 in Figure 2). The term "analyte modulating constituent" is used herein according to art accepted terminology and refers to a constituent that typically forms a membrane on the sensor that operates to modulate the diffusion of one or more analytes, such as glucose, through the constituent. In certain embodiments of the invention, the analyte modulating constituent is an analyte-limiting membrane which operates to prevent or restrict the diffusion of one or more analytes, such as glucose, through the constituents. In other embodiments of the invention, the analyte-modulating constituent operates to facilitate the diffusion of one or more analytes, through the constituents. Optionally such analyte modulating constituents can be formed to prevent or restrict the diffusion of one type of molecule through the constituent (e.g. glucose), while at the same time allowing or even facilitating the diffusion of other types of molecules through the constituent (e.g. O₂).

With respect to glucose sensors, in known enzyme electrodes, glucose and oxygen from blood, as well as some interferents, such as ascorbic acid and uric acid, diffuse through a primary membrane of the sensor. As the glucose, oxygen and interferents reach the analyte sensing constituent, an enzyme, such as glucose oxidase, catalyzes the conversion of glucose to hydrogen peroxide and gluconolactone. The hydrogen peroxide may diffuse back through the analyte modulating constituent, or it may diffuse to an electrode where it can be reacted to form oxygen and a proton to produce a current that is proportional to the glucose concentration. The sensor membrane assembly serves several functions, including selectively allowing the passage of glucose therethrough. In this context, an illustrative analyte modulating constituent is a semi-permeable membrane which permits passage of water, oxygen and at least one selective analyte and which has the ability to absorb water, the membrane having a water soluble, hydrophilic polymer.

A variety of illustrative analyte modulating compositions are known in the art and are described for example in U.S. Patent Nos. 6,319,540, 5,882,494, 5,786,439 5,777,060, 5,771,868 and 5,391,250, the disclosures of each being incorporated herein by reference. The hydrogels described therein are particularly useful with a variety of implantable devices for which it is advantageous to provide a surrounding water constituent. In some embodiments of the invention, the analyte modulating composition includes PDMS. In certain embodiments of the invention, the analyte modulating constituent includes an agent selected for its ability to crosslink a siloxane moiety present in a proximal constituent. In closely related embodiments of the invention, the adhesion promoting constituent includes an agent selected for its ability to crosslink an amine or carboxyl moiety of a protein present in a proximal constituent.

### COVER CONSTITUENT

The electrochemical sensors of the invention include one or more cover constituents which are typically electrically insulating protective constituents (see, e.g. element 106 in Figure 2). Typically, such cover constituents can be in the form of a coating, sheath or tube and are disposed on at least a portion of the analyte modulating constituent. Acceptable polymer coatings for use as the insulating protective cover constituent can include, but are not limited to, non-toxic biocompatible polymers such as silicone compounds, polyimides, biocompatible solder masks, epoxy acrylate copolymers, or the like. Further, these coatings can be photo-imageable to facilitate photolithographic forming of apertures through to the conductive constituent. A typical cover constituent comprises spun on silicone. As is known in the art, this constituent can be a commercially available RTV (room temperature vulcanized) silicone composition. A typical chemistry in this context is polydimethyl siloxane (acetoxy based).

## Claims

1. An analyte sensor system comprising:
(a) an amperometric glucose sensor (12; 100) that is implanted in vivo, comprising:
an elongated flexible base layer (102);
a conductive layer (104) disposed on the base layer;
an analyte sensing layer (110) disposed on the conductive layer;
an analyte modulating layer (112) disposed on the analyte sensing layer;
(b) a sensor input operatively coupled to the amperometric glucose sensor and capable of receiving signals from the amperometric glucose sensor; and
(c) a processor coupled to the sensor input, wherein the processor is capable of analyzing and/or characterizing one or more signals received from the amperometric glucose sensor; and
(d) a multiple-conductor electrical lead for electrically connecting elements in the analyte sensor system together, comprising:
a central core; and
at least one ribbon cable coiled around the central core along a length of the central core, the at least one ribbon cable comprising a plurality of separate electrical conductors coupled together along their lengths in series and electrically insulated from one another with an insulating material; wherein the conductive layer comprises a plurality of working, counter and reference electrodes (20, 360) that are grouped together as a unit and positionally distributed in a repeating pattern of units.

2. The analyte sensor system of claim 1, wherein the multiple-conductor electrical lead comprises a first ribbon cable coiled around the central core and one or more further ribbon cable layers coiled around the first ribbon cable.

3. The analyte sensor system of claim 1, wherein an electrical conductor in the ribbon cable comprises a noble metal composition.

4. The analyte sensor system of claim 1, wherein the central core of the multiple-conductor electrical lead comprises:
a flexible polymeric composition;
a stainless steel composition; or
a shape memory nickel titanium composition

5. The analyte sensor system of claim 1, wherein the sensor (12; 100) is adapted to function using an electrical signal whose polarity is fixed and whose amplitude remains constant with respect to time.

6. The analyte sensor system of claim 1, further comprising:
a flexible sensor substrate; and
a contact pad (350);
wherein the contact pad and the implanted sensor (12; 100) are operatively coupled to the flexible sensor substrate in a configuration that facilitates sensor function as the implanted sensor twists and bends in response to movement of a user.

7. The analyte sensor system of claim 1, wherein the working electrode, the counter electrode and the reference electrode (20; 360) of an amperometric sensor (12; 100) are positionally distributed on the conductive layer (104) in a parallel configuration arranged so that a first electrode is disposed in a region on a first edge of the elongated base layer (102); a second electrode is disposed in a region on an opposite edge of the elongated base layer; and a third electrode is disposed in a region of the elongated base layer that is between the first electrode and the second electrode.

8. The analyte sensor system of claim 1, wherein the working electrode, the counter electrode and the reference electrode (20; 360) are positionally distributed on conductive layer (104) in a configuration arranged so that the working electrode is disposed in a region on a first edge of the elongated base layer (102); the counter electrode is disposed in a region on an opposite edge of the elongated base layer (102); and the reference electrode is disposed in a region of the elongated base layer that between the working electrode and the counter electrode.

## Patentansprüche

1. Analyt-Sensorsystem mit:
(a) einem amphoterischen Glucosesensor (12; 100), der in vivo implantiert ist, mit:
einer langgestreckten flexiblen Basisschicht (102);
einer leitfähigen Schicht (104), angeordnet auf der Basisschicht;
einer Analyt-Sensorschicht (110), angeordnet auf der leitfähigen Schicht;
einer Analyt-Modulierschicht (112), angeordnet auf der Analyt-Sensorschicht;
(b) einem Sensoreingang, der betriebsmäßig mit dem amphoterischen Glucosesensor gekoppelt und befähigt ist, Signale von dem amphoterischen Glucosesensor zu empfangen; und
(c) einem Prozessor, der mit dem Sensoreingang gekoppelt ist, wobei der Prozessor befähigt ist, ein oder mehrere Signal(e) von dem amphoterischen Glucosesensor zu analysieren und/oder zu charakterisieren; und
(d) einem elektrischen Leiter mit mehrfachen Strängen zum elektrischen Verbinden der Elemente in dem Analyt-Sensorsystem miteinander, aufweisend:
einen zentralen Kern; und
wenigstens ein Bandkabel, das um den zentralen Kern entlang einer Länge des zentralen Kerns herumgespult ist, wobei das wenigstens eine Bandkabel eine Mehrzahl von separaten elektrischen Leitern aufweist, die entlang ihrer Längen in Serie zusammengeschaltet und elektrisch voneinander mit Hilfe von einem Isoliermaterial isoliert sind;
wobei die leitfähige Schicht eine Mehrzahl von Arbeits-, Gegen- und Bezugs-Elektroden (20, 360) aufweist, die als eine Einheit zusammengefasst und positionsmäßig in einem sich wiederholenden Muster von Einheiten angeordnet sind.

2. Analyt-Sensorsystem nach Anspruch 1, bei dem der elektrische Leiter mit mehrfachen Strängen ein erstes Bandkabel umfasst, das um den zentralen Kern und eine oder mehrere weitere Bandkabelschichten herumgespult ist, die um die erste Bandkabelschicht herumgespult sind.

3. Analyt-Sensorsystem nach Anspruch 1, bei dem der elektrische Leiter in dem Bandkabel eine Edelmetallzusammensetzung aufweist.

4. Analyt-Sensorsystem nach Anspruch 1, bei dem der zentrale Kern des elektrischen Leiters mit mehrfachen Strängen aufweist:
eine flexible polymerische Zusammensetzung;
eine rostfreie Stahlzusammensetzung; oder
eine Nickel-Titan-Zusammensetzung mit Formgedächtnis.

5. Analyt-Sensorsystem nach Anspruch 1, wobei der Sensor (12; 100) zur Verwendung eines elektrischen Signals eingerichtet ist, dessen Polarität festgelegt ist und dessen Amplitude in Bezug auf die Zeit konstant bleibt.

6. Analyt-Sensorsystem nach Anspruch 1, weiterhin mit:
einem flexiblen Sensorsubstrat; und
einem Kontaktkissen (350);
wobei das Kontaktkissen und der implantierte Sensor (12; 100) betriebsmäßig mit dem flexiblen Sensorsubstrat in einer Konfiguration gekoppelt sind, die die Sensorfunktion erleichtert, wenn der implantierte Sensor verdreht und verbogen wird als Reaktion auf Bewegungen eines Nutzers.

7. Analyt-Sensorsystem nach Anspruch 1, indem die Arbeitselektrode, die Gegenelektrode und die Bezugselektrode (20; 360) eines amphoterischen Sensors (12; 100) positionsmäßig verteilt sind auf der leitfähigen Schicht (104) in einer parallelen Konfiguration, die so arrangiert ist, dass eine erste Elektrode angeordnet ist in einer Region auf einem ersten Rand der langgestreckten Basisschicht (102); eine zweite Elektrode ist angeordnet in einer Region auf dem gegenüberliegenden Rand der langgestreckten Basisschicht; und eine dritte Elektrode ist angeordnet in einer Region der langgestreckten Basisschicht, die zwischen der ersten Elektrode und der zweiten Elektrode angeordnet ist.

8. Analyt-Sensorsystem nach Anspruch 1, bei dem die Arbeitselektrode, die Gegenelektrode und die Referenzelektrode (20; 360) positionsmäßig verteilt sind auf der leitfähigen Schicht (104) in einer Konfiguration, die so arrangiert ist, dass die Arbeitselektrode angeordnet ist in einer Region auf einem ersten Rand der langgestreckten Basisschicht (102); die Gegenelektrode ist angeordnet in einer Region auf einem gegenüberliegenden Rand der langgestreckten Basisschicht (102); und die Referenzelektrode ist angeordnet in einer Region der langgestreckten Basisschicht zwischen der Arbeitselektrode und der Gegenelektrode.

## Revendications

1. Système de capteur d'analyte comprenant :
(a) un capteur de glucose ampérométrique (12 ; 100) qui est implanté in vivo, comprenant :
une couche de base flexible allongée (102) ;
une couche conductrice (104) disposée sur la couche de base ;
une couche de détection d'analyte (110) disposée sur la couche conductrice ;
une couche de modulation d'analyte (112) disposée sur la couche de détection d'analyte ;
(b) une entrée de capteur couplée de manière opérationnelle au capteur de glucose ampérométrique et capable de recevoir des signaux provenant du capteur de glucose ampérométrique ; et
(c) un processeur couplé à l'entrée de capteur, dans lequel le processeur est capable d'analyser et/ou de caractériser un ou plusieurs signaux reçus du capteur de glucose ampérométrique ; et
(d) un fil électrique à conducteurs multiples pour relier électriquement ensemble des éléments dans le système de capteur d'analyte, comprenant :
une âme centrale ; et
au moins un câble à ruban enroulé autour de l'âme centrale le long d'une longueur de l'âme centrale, le au moins un câble à ruban comprenant une pluralité de conducteurs électriques séparés couplés ensemble en série le long de leurs longueurs et électriquement isolés les uns des autres avec un matériau isolant ;
dans lequel la couche conductrice comprend une pluralité d'électrodes de travail, de contre-électrodes et d'électrodes de référence (20, 360) qui sont regroupées ensemble comme une unité et réparties par position selon un motif répétitif d'unités.

2. Système de capteur d'analyte selon la revendication 1, dans lequel le fil électrique à conducteurs multiples comprend un premier câble à ruban enroulé autour de l'âme centrale et une ou plusieurs couches de câble à ruban supplémentaires autour du premier câble à ruban.

3. Système de capteur d'analyte selon la revendication 1, dans lequel un conducteur électrique dans le câble à ruban comprend une composition de métal noble.

4. Système de capteur d'analyte selon la revendication 1, dans lequel l'âme centrale du fil électrique à conducteurs multiples comprend :
une composition polymère flexible ;
une composition d'acier inoxydable ; ou
une composition de nickel-titane à mémoire de forme.

5. Système de capteur d'analyte selon la revendication 1, dans lequel le capteur (12 ; 100) est adapté pour fonctionner en utilisant un signal électrique dont la polarité est fixe et dont l'amplitude reste constante par rapport au temps

6. Système de capteur d'analyte selon la revendication 1, comprenant en outre :
un substrat de capteur flexible ; et
un plot de contact (350) ;
dans lequel le plot de contact et le capteur implanté (12 ; 100) sont couplés de manière opérationnelle au substrat de capteur flexible dans une configuration qui facilite la fonction de capteur lorsque le capteur implanté se tort et fléchit en réponse à un mouvement d'un utilisateur.

7. Système de capteur d'analyte selon la revendication 1, dans lequel l'électrode de travail, la contre-électrode et l'électrode de référence (20 ; 360) d'un capteur ampérométrique (12 ; 100) sont réparties par position sur la couche conductrice (104) dans une configuration parallèle agencée de sorte qu'une première électrode est disposée dans une zone sur un premier bord de la couche de base allongée (102) ; une deuxième électrode est disposée dans une zone sur un bord opposé de la couche de base allongée ; et une troisième électrode est disposée dans une zone de la couche de base allongée qui est entre la première électrode et la deuxième électrode.

8. Système de capteur d'analyte selon la revendication 1, dans lequel l'électrode de travail, la contre-électrode et l'électrode de référence (20 ; 360) sont réparties par position sur la couche conductrice (104) dans une configuration agencée de sorte que l'électrode de travail est disposée dans une zone sur un premier bord de la couche de base allongée (102) ; la contre-électrode est disposée dans une zone sur un bord opposé de couche de base allongée (102) ; et l'électrode de référence est disposée dans une zone de la couche de base allongée qui est entre l'électrode de travail et la contre-électrode.
